(19) Europäisches Patentamt
European Patent Office
Office européen des brevets

(11) Numéro de publication : **0 394 118 B1**

# (12) FASCICULE DE BREVET EUROPEEN

(45) Date de publication du fascicule du brevet : **01.06.94 Bulletin 94/22**

(51) Int. Cl.$^5$ : **C07C 229/30,** A61K 31/195, C07C 247/08, C07F 7/10

(21) Numéro de dépôt : **90401033.7**

(22) Date de dépôt : **17.04.90**

## (54) Nouveaux dérivés insaturés de l'acide 2,6-amino heptanedioique, leur procédé de préparation et leur application comme médicaments.

(30) Priorité : **18.04.89 FR 8905108**

(43) Date de publication de la demande : **24.10.90 Bulletin 90/43**

(45) Mention de la délivrance du brevet : **01.06.94 Bulletin 94/22**

(84) Etats contractants désignés : **AT BE CH DE DK ES FR GB GR IT LI LU NL SE**

(56) Documents cités :
**EP-A- 0 284 461**
**FR-A- 2 566 410**
**GB-A- 2 104 887**
**JOURNAL OF MEDICINAL CHEMISTRY, vol. 29, no. 6, juin 1986, pages 1023-1030, American Chemical Society, Washington, DC, US; J.-M. GIRODEAU et al.: "The lysine pathway as a target for a new genera of synthetic antibacterial antibiotics?"**

(56) Documents cités :
**TETRAHEDRON LETTERS, vol. 26, no. 26, 1985, pages 3115-3118, Pergamon Press Ltd, Oxford, GB; K. AGOURIDAS et al.: "Synthesis of protected vinylic amino acids by intermolecular Lewis acid catalyzed ene reactions"**

(73) Titulaire : **ROUSSEL-UCLAF**
**35, Boulevard des Invalides**
**F-75007 Paris (FR)**

(72) Inventeur : **Agouridas, Constantin**
**67, Quai d'Orsay**
**F-75007 Paris (FR)**
Inventeur : **Tessot, Nicole**
**Hameau de la Dimeresse-Messy**
**F-77410 Claye-Souilly (FR)**
Inventeur : **Martel, Annie**
**40, rue du Regard - Bât. 1**
**F-94260 Fresnes (FR)**

(74) Mandataire : **Tonnellier, Marie-José et al**
**111, route de Noisy**
**B.P. no 9**
**F-93230 Romainville (FR)**

EP 0 394 118 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés insaturés de l'acide 2,6-diamino heptanedioïque, leur procédé de préparation et leur application comme médicaments.

On connaissait déjà des dérivés de l'acide diaminopimélique et notamment l'acide 2,6-diamino 4-méthylène 1,7-heptanedioïque doué d'intéressantes propriétés antibactérienne (cf J. Med. Chem. 29 1023-1030 (1986)).

L'invention a pour objet les composés de formule générale (I) :

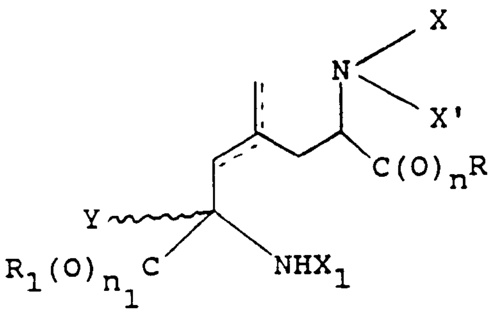

(I)

dans lesquels,

- les traits pointillés représentent une double liaison éventuelle endo ou exo,
- Y représente un radical alkyle, alcényle ou alcynyle renfermant de 2 à 18 atomes de carbone, ou un radical alkyle renfermant de 1 à 18 atomes de carbone substitué par un ou plusieurs atomes d'halogène,
- X, X' et $X_1$ identiques ou différents représentent un atome d'hydrogène, un radical alkyle, alcényle ou alcynyle renfermant jusqu'à 8 atomes de carbone, un radical aryle renfermant jusqu'à 14 atomes de carbone, un radical aralkyle renfermant jusqu'à 18 atomes de carbone, ou un radical acyle dérivé d'un acide gras et/ou d'un acide aminé alpha et/ou oméga,

et soit n et/ou $n_1$ représente le nombre 1 et R et/ou $R_1$ représente le reste d'une amine ou d'un acide aminé alpha ou oméga,

soit n et/ou $n_1$ représente le nombre 2 et R et $R_1$, représentent un atome d'hydrogène, un radical alkyle renfermant jusqu'à 8 atomes de carbone ou un radical aryle renfermant jusqu'à 14 atomes de carbone, ou un radical aralkyle renfermant jusqu'à 18 atomes de carbone, ou un radical

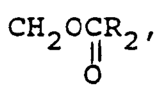

dans lesquels $R_2$ représente un radical alkyle renfermant jusqu'à 8 atomes de carbone, ou un radical aryle renfermant jusqu'à 14 atomes de carbone, ainsi que leurs sels avec les acides organiques ou minéraux ou avec les bases.

Lorsque Y représente un radical alkyle, il s'agit de préférence du radical éthyle, propyle, isopropyle ou butyle ou d'un radical insaturé comme le radical vinyle, allyle, éthynyle ou propynyle.

Lorsque Y représente un radical alkyle substitué par un ou plusieurs atomes d'halogène, il s'agit de préférence d'un radical alkyle substitué par un ou plusieurs atomes de fluor ou de chlore, par exemple, les radicaux $-CHF_2$-, $-CH_2F$, $-CHCl_2$, $-CH_2Cl$.

Lorsque X, X', $X_1$, R, $R_1$ ou $R_2$ représente un radical alkyle, il s'agit de préférence du radical méthyle, éthyle, propyle, isopropyle ou butyle.

Lorsque X, X' ou $X_1$ représente un radical alcényle, il s'agit de préférence d'un radical vinyle ou allyle.

Lorsque X, X' ou $X_1$ représente un radical alcynyle, il s'agit de préférence d'un radical éthynyle ou propynyle.

Lorsque X, X', $X_1$, R, $R_1$ ou $R_2$ représente un radical aryle, il s'agit de préférence du radical phényle.

Lorsque X, X', $X_1$, R ou $R_1$ représente un radical aralkyle, il s'agit de préférence du radical benzyle.

$R_2$ représente de préférence un radical méthyle, éthyle, n-propyle ou phényle.

Lorsque dans la définition des substituants, il est question d'acide gras, il s'agit de préférence d'acide aliphatique saturé ou insaturé renfermant de 6 à 24 atomes de carbone et de préférence de 12 à 22 atomes de carbone ; on peut citer par exemple les acides stéarique, palmitique, laurique, caprylique, myristique, alpha

ou gamma linolénique, linoléique, arachidonique, docosapentaénoïque, ou adamantane carboxylique.

Lorsque dans la définition des substituants, il est question d'amines, il s'agit de préférence de la benzylamine, éventuellement substitué, la méthylamine, la diméthylamine ou tout autre amine secondaire ou tertiaire.

Lorsque dans la définition des substituants, il est question d'acides aminés, il s'agit de préférence d'un acide aminé choisi dans le groupe constitué par Ala, Val, Ival, Leu, Ile, Asp, Asn, Glu, Gln, Ser, Thr, Cys, Met Lys, Arg, Phe, Tyr, Trp, His et Pro, Nva, Nle, Hyp, Orn, ces acides étant sous la forme D ou L ainsi que par Sar et Gly, tous les acides précédemment nommés pouvant être N-acylés ou N-alkylés.

On admettra par convention que les symboles des acides alpha-amino carboxyliques représentent ces acides sous leur configuration D ou L (par exemple, le terme Ala signifie Alanine sous forme D ou sous forme L).

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléïque, fumarique, succinique, tartrique, citrique, oxalique, glyoxylique, aspartique, alcanesulfoniques tels que les acides méthane ou éthanesulfoniques, arylsulfoniques, tels que les acides benzène ou paratoluènesulfoniques et arylcarboxyliques.

Les sels des dérivés de l'invention peuvent être formés avec des bases organiques et minérales. Parmi les bases minérales, on peut citer les hydroxydes alcalins et alcalino-terreux, tels que par exemple, les hydroxydes de sodium, de potassium, de lithium et de calcium, l'hydroxyde de magnésium ou d'ammonium. Parmi les bases organiques, on peut citer les amines alkylées substituées ou non substituées, telles que par exemple, la triméthylamine, la méthylamine, la propylamine, la N,N-diméthyléthanolamine ou la tris(hydroxyméthyl) méthylamine ; on peut citer également des acides aminés basiques tels que, par exemple, la lysine ou l'arginine ; on peut citer encore d'autres bases telles que, par exemple, la glucosamine ou la procaïne.

Parmi les composés préférés de l'invention, on peut citer les composés de formule (I), dans lesquels les traits pointillés représentent une double liaison exo, ceux dans lesquels X′ représente un atome d'hydrogène, ceux dans lesquels X, X′ et $X_1$ représentent un atome d'hydrogène, ceux dans lesquels X′ représente un atome d'hydrogène ou un radical méthyle, X représente un radical méthyle ou benzyle et $X_1$ représente un atome d'hydrogène et ceux dans lesquels R et $R_1$ représentent chacun un atome d'hydrogène, ainsi que leurs sels d'addition avec les acides organiques ou minéraux ou avec les bases.

Parmi les composés préférés de l'invention, on peut citer les composés dans lesquels Y représente un radical acétylénique, ceux dans lesquels Y représente un radical éthylénique ou encore ceux dans lesquels Y représente un radical éthyle.

L'invention a tout particulièrement pour objet les composés dont la préparation est donnée ci-après dans la partie expérimentale, comme par exemple les composés des exemples 4, 7 et 10.

Les dérivés, objet de la présente invention, possèdent de très intéressantes propriétés pharmacologiques, ils sont doués notamment d'intéressantes propriétés anti-bactériennes.

Ces propriétés sont illustrées plus loin dans la partie expérimentale.

Ces propriétés justifient l'utilisation des produits de l'invention ainsi que de leurs sels, à titre de médicaments.

La présente demande a ainsi également pour objet l'application, à titre de médicaments, des dérivés de formule (I) ainsi que de leurs sels d'addition pharmaceutiquement acceptables.

Les médicaments selon l'invention trouvent leur emploi, par exemple, dans l'antibiothérapie vis-à-vis des germes bactériens, des levures, des champignons (candida albicans ...) dans la thérapie anti-virale, et dans la chimiothérapie anti-cancéreuse, seuls ou en association, et enfin comme adjuvants à une antibiothérapie classique ou à la vaccination.

L'invention a enfin pour objet les compositions pharmaceutiques qui renferment au moins un dérivé précité ou l'un de ses sels d'addition avec les acides ou les bases pharmaceutiquement acceptables, à titre de principe actif.

A titre de médicaments, les dérivés répondant à la formule (I) et leurs sels d'addition avec les acides ou les bases pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme, par exemple, les comprimés, simples ou dragéifiés, les gélules, les granulés, les suppositoires, les préparations injectables, elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants

ou émulsifiants, les conservateurs.

L'invention a également pour objet un procédé de préparation caractérisé en ce que l'on soumet un composé de formule générale (II) :

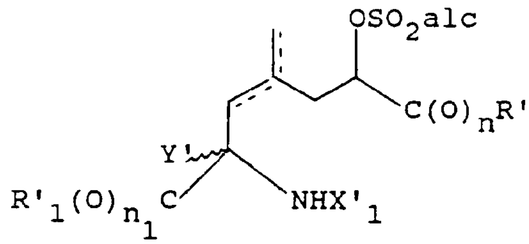

(II)

dans laquelle alc représente un radical alkyle renfermant de 1 à 8 atomes de carbone éventuellement substitué par un ou plusieurs atome d'halogène, n, $n_1$ et les traits pointillés conservent la même signification que précédemment, Y' représente soit Y, soit un précurseur de Y, $X'_1$, R' et R'1 ont la même signification que $X_1$, R et $R_1$ à l'exception de l'hydrogène, à l'action d'un réactif capable de fournir un précurseur de la fonction amino ou d'une amine de formule

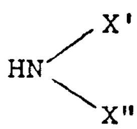

dans laquelle X' a la signification indiquée précédemment et X" a la signification indiquée pour X à l'exception de l'hydrodrogène pour obtenir, le cas échéant, après traitement le composé de formule (I) que l'on soumet, si désiré, à la totalité ou à une partie seulement des opérations suivantes dans un ordre quelconque :

- déprotection des fonctions amines,
- fonctionnalisation des fonctions amines,
- hydrolyse des fonctions esters,
- traitement de Y' pour obtenir Y,
- réduction totale ou sélective du substituant Y lorsque celui-ci est insaturé,
- salification.

Dans un mode de réalisation préféré,

- alc représente un radical méthyle, éthyle ou n-propyle ou un radical $CF_3$,
- le réactif capable de fournir un précurseur de la fonction amino est l'azoture de sodium, le phtalimide de potassium ou l'hydroxylamine.

L'obtention de l'amine est effectuée par des méthodes connues de l'homme de métier par exemple par hydrolyse dans le cas du phtalamide.

- l'agent d'hydrolyse du phtalimide dans ce cas peut être une base minérale suivie d'une hydrolyse acide ou l'hydrazine,
- la déprotection de la fonction amine est réalisée de préférence par action d'un acide minéral dilué tel que l'acide chlorhydrique, ou d'un acide organique tel que l'acide trifluoroacétique,
- l'hydrolyse des fonctions esters est réalisée de préférence par saponification à l'aide d'une base minérale comme la soude ou la potasse, suivie éventuellement d'un traitement par une résine acide,
- la salification est réalisée par addition d'acide ou de base au milieu réactionnel.

L'invention a tout particulièrement pour objet un procédé caractérisé en ce que l'on soumet un composé de formule (II) défini comme précédemment à l'action de l'azoture de sodium pour obtenir le composé de formule (III) :

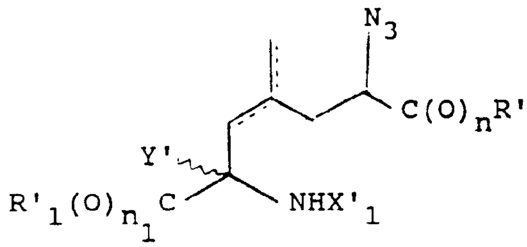

(III)

que l'on soumet à l'action d'un agent de réduction du groupement $N_3$, pour obtenir le composé de formule (IV) :

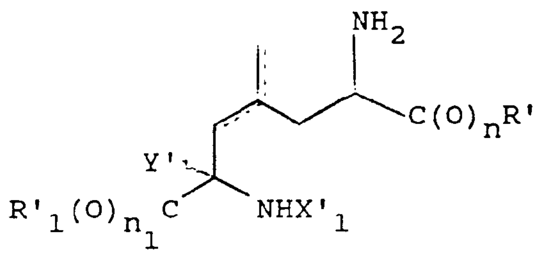

(IV)

que l'on soumet à la totalité ou à une partie seulement des opérations mentionnées précédemment.

Dans un mode de réalisation préféré :

- l'agent de réduction que l'on fait réagir avec le composé de formule (III) est la triphénylphosphine, suivie d'une hydrolyse acide, on peut également procéder par hydrogénation catalytique, par exemple en présence du palladium sur charbon actif empoisonné par la quinoléïne.

L'invention a plus particulièrement pour objet un procédé caractérisé en ce que l'on soumet un composé de formule ($II_A$) :

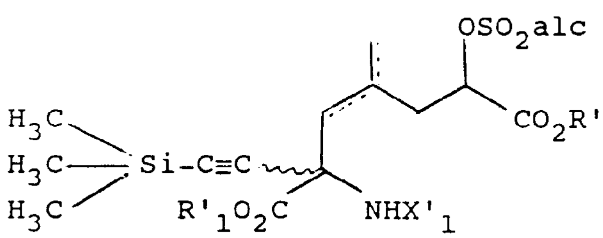

($II_A$)

soit à l'action de l'azoture de sodium pour obtenir le composé de formule ($III_A$) :

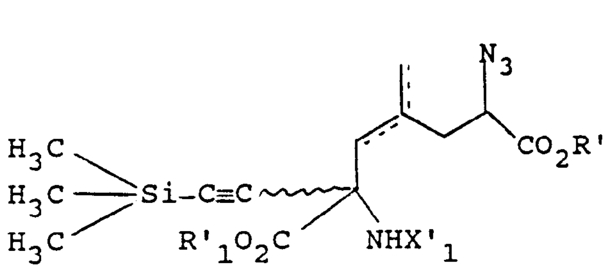

($III_A$)

que l'on soumet à l'action d'un agent de réduction de l'azoture pour obtenir le composé de formule ($IV_A$) :

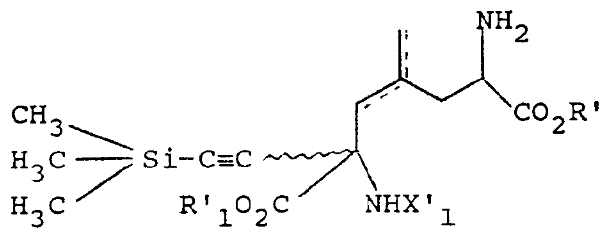

$(IV_A)$

soit à l'action d'une amine de formule

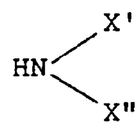

dans laquelle X' et X" ont la signification indiquée précédemment pour obtenir le composé de formule ($IV_B$) :

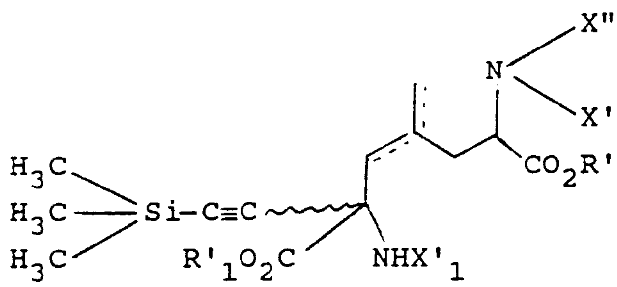

$(IV_B)$

puis ou bien soumet le composé ($IV_A$) ou ($IV_B$) à l'action d'un agent de clivage sélectif des fonctions esters et du radical triméthylsilyle pour obtenir le composé de formule ($I_A$) :

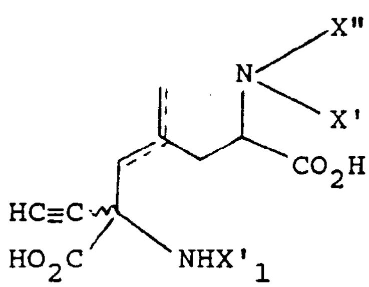

$(I_A)$

que le cas échéant l'on soumet à l'action d'un agent de clivage du groupement protecteur d'amine pour obtenir le composé de formule ($I_B$) :

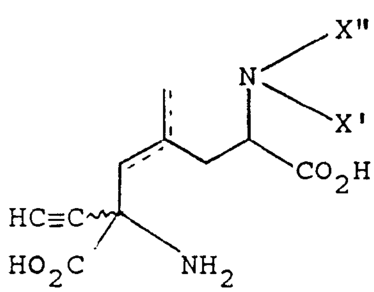

$(I_B)$

ou bien soumet le composé de formule ($IV_A$) ou ($IV_B$) à l'action d'un agent de clivage du triméthylsilyle pour obtenir le composé de formule ($I_C$) :

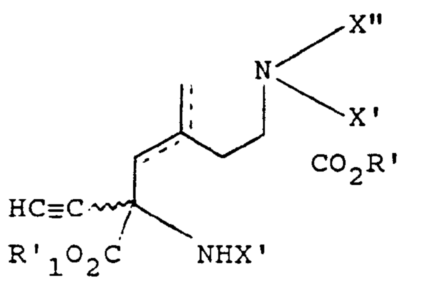

($I_C$)

puis l'on soumet si désiré, le compose de formule ($I_A$), ($I_B$) ou ($I_C$) à l'action d'un agent de réduction partielle pour obtenir le composé de formule ($I_D$) correspondant dans lequel Y représente un radical éthényle, ou à l'action d'agent de réduction totale de la triple liaison pour obtenir le composé de formule ($I_E$) correspondant dans lequel Y est un radical éthyle,
puis le cas échéant soumet les composés de formule (I) ainsi obtenus à la totalité ou à une partie seulement des opérations mentionnées précédemment.

Dans un mode de réalisation préféré du procédé :
- l'agent de clivage du groupement triméthylsilyle est le fluorure de potassium ou le fluorure de tétrabutylammonium.

Les produits de formule (I) dans laquelle R et/ou $R_1$ représentent le reste d'une amine peuvent être obtenus par amidification des fonctions acides du produit de formule ($I_A$), l'amidification est réalisée en présence d'agents de condensation comme le dicyclohexylcarbodiimide, le N, N'-carbonyldiimidazole, ou des amides bis-alkyliques d'acides sulfurés tels que le N, N'-sulfinyl-bis(diméthylamine), $SO[N(CH_3)_3]_2$, ou encore par la formation d'anhydride mixte avec le chloroformiate d'isobutyle.

Certains produits de départs de formule (II) sont décrits et revendiqués dans la demande de brevet européen 88 402741.8 déposée le 2.11.1988, leur préparation est rappelée ci-après.

Les composés de formule (II) et ($II_A$) peuvent être préparés par exemple, selon les procédés décrits ci-après dans la partie expérimentale, par exemple, selon le schéma :

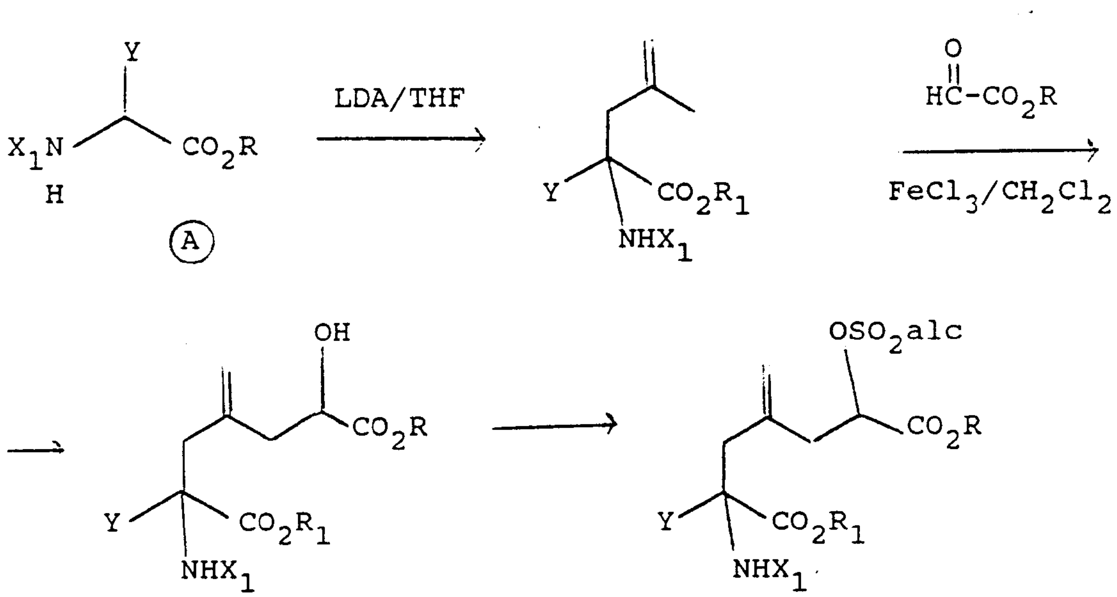


Les composés de formule (II) dans lesquels Y' représente un radical triméthylsilyle alkynyle sont nouveaux et sont en eux-mêmes un objet de l'invention.

Les produits A sont connus et peuvent par exemple être préparés selon le procédé décrit par USP 4088667 ou dans Tet. Lett. CASARA et al. 1978 p. 1581 et suivantes.

Les composés de formule (III) et ($III_A$) sont nouveaux et sont en eux-mêmes un objet de la présente invention.

Les exemples suivants illustrent l'invention sans toutefois la limiter.

**EXEMPLE 1 : Acide 6-amino 2-éthynyl 2-[(méthoxycarbonyl) amino] 4-méthylène heptanedioïque**

STADE A : 6-azido 2-[(méthoxycarbonyl) amino] 4-méthylène 2-[2-(triméthylsilyl) éthynyl] heptanedioate de 7-éthyle 1-méthyle.

On ajoute 0,498 g d'azoture de sodium dans une solution renfermant 3,32 g de 6-(méthanesulfonyloxy) 2-[(méthoxycarbonyl) amino 4-méthylène 2-[2-(triméthyl) éthynyl] heptanedioate de 7-éthyle 1-méthyle préparé comme indiqué à la suite de l'exemple 1, et 30 $cm^3$ de diméthylformamide. On maintient le mélange réactionnel, sous agitation, pendant 16 heures. On filtre, dilue à l'eau, extrait à l'éther, sèche et évapore à sec. On obtient 3,7 g de produit que l'on purifie par chromatographie sur silice en éluant par le mélange cyclohexaneacétate d'éthyle (6-4). On obtient 2,28 g de produit recherché Rf = 0,35.

STADE B : 6-amino 2-[(méthoxycarbonyl) amino] 4-méthylène 2-[2-triméthylsilyl) éthynyl] heptanedioate de 7-éthyle 1-méthyle.

On dissout 519 mg du produit obtenu au stade précédent dans 6 $cm^3$ de tétrahydrofuranne. On ajoute 341 mg de triphénylphosphine, agite 16 heures à la température ambiante. On ajoute 0,65 $cm^3$ d'eau et agite pendant 30 heures. On dilue à l'éther le produit obtenu, extrait avec une solution normale d'acide chlorhydrique, lave la phase aqueuse à l'éther. On ajoute du bicarbonate de soude et extrait à l'éther. On lave, sèche et amène à sec. On obtient 433 mg de produit recherché. Rf = 0,4 (éluant acétate d'éthyle).

STADE C : 6-amino 2-éthynyl 2-[(méthoxycarbonyl) amino] 4-méthylène heptanedioïque.

On dissout 1,23 g de produit préparé comme au stade précédent dans 12 $cm^3$ d'éthanol. On refroidit à + 4°C et ajoute 5 $cm^3$ d'une solution de soude 2N. On laisse revenir à température ambiante la solution obtenue et maintient sous agitation pendant 38 heures. On ajoute de la résine Dowex 50 W x 8. On filtre, rince à l'eau, élue à l'ammoniaque diluée et évapore à sec. On obtient 709 mg de produit que l'on chromatographie sur silice en éluant par le mélange éthanol-ammoniaque (8-2). On obtient 600 mg de produit recherché. Rf = 0,2.

PREPARATION 1 : 6-(méthanesulfonyloxy) 2-[(méthoxycarbonyl) amino] 4-méthylène 2-[2-triméthylsilyl) éthynyl] heptanedioate de 7-éthyle 1-méthyle.

STADE A : 2-[(méthoxycarbonyl) amino] 4-méthylène 2-[2-(triméthylsilyl) éthynyl] 4-pentènoate de méthyle.

On refroidit à -60°C une solution renfermant 183,4 g de diisopropylamine dans 5570 $cm^3$ de tétrahydrofuranne. On introduit entre -50°C et -60°C, 1135 $cm^3$ de butyllithium à 15 % dans l'hexane. On laisse remonter la température à 0°C agite pendant 15 minutes, refroidit à -66°/-65°C et ajoute en 50 minutes une solution renfermant 147 g de 2-[(méthoxycarbonyl) amino] 4-(triméthylsilyl) 3-butynoate de méthyle (préparé comme il est indiqué pour l'analogue carbonate d'éthyle dans Tetrahedron Letters n° 18 p 1581 (1978)), dans 1100 $cm^3$ de tétrahydrofuranne. On agite pendant 45 minutes à -65°/-70°C et ajoute à cette température 558,6 $cm^3$ d'hexaneméthylphosphotriamide. On agite pendant 15 minutes à cette température et ajoute 81,6 g de bromure de méthallyle en solution dans 1100 $cm^3$ de tétrahydrofuranne. On agite pendant 2 heures à -67°/-70°C et ajoute 186 $cm^3$ d'acide acétique dilué 10 fois dans le tétrahydrofuranne. On laisse revenir à +20°C, filtre, lave au tétrahydrofuranne et distille à sec sous pression réduite. On reprend l'extrait sec au chlorure de méthylène et lave à l'eau. On sèche les phases organiques, les lave au chlorure de méthylène et les amène à sec. On obtient 587,5 g de produit brut que l'on chromatographie sur silice, en éluant par le mélange cyclohexane acétate d'éthyle (7-3). On obtient le produit recherché. Rf = 0,4.

STADE B : 6-hydroxy 2-[(méthoxycarbonyl) amino] 4-méthylène 2-[2-(triméthylsilyl) éthynyl] heptanedioate de 7-éthyle 1-methyle.

On met en suspension 9,72 g de chlorure ferrique dans 50 $cm^3$ de chlorure de méthylène, en présence de siliporite. On refroidit à 0°C et introduit en 30 minutes 3,1 g de glyoxylate d'éthyle en solution dans 40 $cm^3$ de chlorure de méthylène. On agite ensuite 30 minutes à la température ambiante, refroidit la solution à -60°C et introduit en 35 minutes 4,5 g de produit préparé au stade précédent, en solution dans 40 $cm^3$ de chlorure

de méthylène. On agite à -60°C le mélange réactionnel pendant 2 heures. On le verse sur un excès de bicarbonate de sodium (le milieu restant basique). On filtre, rince, lave à l'eau, sèche et évapore à sec. On obtient 6,4 g de produit que l'on purifie par chromatographie sur silice en éluant par le mélange cyclohexane-acétate d'éthyle (5-5). On obtient 3,64 g de produit recherché. Rf = 0,37.

STADE C : 6-(méthanesulfonyloxy) 2-[(méthoxycarbonyl) amino] 4-méthylène 2-[2-triméthylsilyl) éthynyl] heptanedioate de 7-éthyle 1-méthyle.

On refroidit à 0°C 8,43 g du produit préparé comme au stade précédent dans 70 $cm^3$ de pyridine. On introduit 1,7 $cm^3$ de chlorure de mésyle. On agite 5 heures à la température ambiante. On évapore la pyridine à la température ambiante sous pression réduite. On reprend le résidu obtenu à l'éther, lave la solution éthérée avec une solution normale d'acide chlorhydrique jusqu'à pH acide, puis lave à l'eau salée jusqu'à neutralité. On sèche la phase organique et évapore à sec à une température inférieure à 30°C. On obtient 11 g de produit recherché que l'on chromatographie en éluant avec le mélange cyclohexane acétate d'éthyle (6-4). On obtient 8,28 g de produit recherché. Rf = 0,35.

**EXEMPLE 2 : Acide 2,6-diamino 2-éthynyl 4-méthylène heptanedioïque**

On chauffe 5 heures à 120°C un mélange comprenant 246 mg de produit obtenu au stade B de l'exemple 1, 5 $cm^3$ de soude 5N. On glace, amène à pH 6 avec de l'acide chlorhydrique concentré et évapore à sec. On fait passer le produit sur une résine échangeuse d'ions Dowex 50 W x 8. On élue à l'eau puis à l'aide d'une solution d'ammoniaque 2N et amène à sec les fractions contenant le produit. On obtient après lyophilisation 85 mg de produit recherché. Rf = 0,24 (éluant : butanolacétate d'éthyle-eau (4-2-2)).

**EXEMPLE 3 : Acide 6-amino 2-éthényl 2-[(méthoxycarbonyl) amino] 4-méthylène heptanedioïque**

STADE A : 6-amino 2-éthynyl 2-[(méthoxycarbonyl) amino] 4-méthylène heptanedioate de 7-éthyle 1-méthyle.

On ajoute 99 mg de fluorure de potassium dans une solution renfermant 338 mg de produit obtenu au stade B de l'exemple 1 dans 4 $cm^3$ de diméthylformamide. On agite à la température ambiante pendant 16 heures. On dilue à l'éther, lave à l'eau, sèche et évapore à sec. On obtient 320 mg de produit que l'on chromatographie sur silice en éluant par le mélange cyclohexane-acétate d'éthyle (7-3)), puis en éluant avec de l'acétate d'éthyle. On obtient 182 mg de produit recherché. Rf = 0,3.

STADE B : 6-amino 2-éthényl 2-[(méthoxycarbonyl) amino] 4-méthylène heptanedioate de 7-éthyle 1-méthyle.

On dissout 494 mg de produit préparé au stade précédent dans 50 $cm^3$ d'éthanol, ajoute 99 microlitres de quinoléïne et 250 mg de palladium à 5 % sur sulfate de baryum. On hydrogène (P = 1200 mm/hg) jusqu'à absorption de 34 $cm^3$. On filtre, rince à l'éthanol et évapore à sec. On obtient 560 mg de produit que l'on purifie par chromatographie sur silice, en éluant à l'acétate d'éthyle. On obtient 433 mg de produit recherché : Rf = 0,3.

STADE C : 6-amino 2-éthynyl 2-[(méthoxycarbonyl) amino] 4-méthylène heptanedioïque.

On dissout 400 mg de produit préparé au stade précédent dans 2,5 $cm^3$ d'éthanol. On glace et introduit 2,5 $cm^3$ de soude 2N. On agite 24 heures à la température ambiante. On ajoute 1 $cm^3$ de lessive de soude et agite à la température ambiante pendant 3 heures. On dilue la solution à l'eau, et ajoute de la résine Dowex 50 X 8, jusqu'à l'obtention de pH 2. On filtre, rince à l'eau, on élue à l'ammoniaque N puis à l'ammoniaque à 40 % et évapore à sec. On obtient 208 mg de produit recherché. Rf = 0,25 (éluant : EtOH - $NH_4OH$ (8-2)).

**EXEMPLE 4 : Acide 2,6-diamino 2-éthényl 4-méthylène heptanedioïque**

On dissout 220 mg de produit obtenu au stade C de l'exem ple 3 dans 2,5 $cm^3$ d'eau et 2,5 $cm^3$ de lessive de soude. On porte à 100°C pendant 5 heures. On laisse revenir à la température ambiante, ajoute de la glace, et acidifie par la résine Dowex 50 W x 8. On filtre, rince à l'eau, puis à l'ammoniaque diluée et évapore à sec. On obtient 190 mg de produit que l'on chromatographie sur silice en éluant dans le mélange éthanol-ammoniaque (8-2). On obtient 76 mg de produit recherché. Rf = 0,5.

**EXEMPLE 5 : 6-amino 2-éthyl 2-[(méthoxycarbonyl) amino] 4-méthylène heptanodioate de 1-méthyle**

STADE A : 6-amino 2-éthyl 2-[(méthoxycarbonyl) amino] 4-méthylène heptanedioate de 7-éthyle 1-méthyle.

On dissout 1,17 g de produit obtenu au stade A de l'exemple 3 dans 100 $cm^3$ d'éthanol. On ajoute 1,1 g de palladium à 5 % sur sulfate de baryum et 0,1 $cm^3$ de quinoléine. On hydrogène jusqu'à saturation. On filtre, rince à l'éthanol et évapore à sec. On obtient 1,32 g de produit que l'on purifie par chromatographie sur silice en éluant avec de l'acétate d'éthyle. On obtient 1,1 g de produit recherché. Rf = 0,35.

STADE B : 6-amino 2-éthyl 2-[(méthoxycarbonyl) amino] 4-méthylène heptanedioate de 1-méthyl.

On dissout 200 mg de produit obtenu au stade A dans 2 $cm^3$ d'éthanol. On ajoute goutte à goutte à la température ambiante 0,55 $cm^3$ de soude N. On maintient sous agitation à la température ambiante pendant 1 h 30. On dilue à l'eau et acidifie à pH 3 par de la résine Dowex 50 W x 8. On filtre, rince à l'eau puis extrait la fraction aminée par lavage de la résine à l'ammoniaque diluée au 1/10. On évapore à sec reprend le résidu à l'eau, filtre et lyophilise. On obtient 131 mg de produit que l'on purifie par chromatographie sur silice en éluant par le mélange chlorure de méthylène méthanol (8/2). Rf = 0,25. On purifie le produit obtenu par passage sur résine. On obtient ainsi le produit recherché (Rt 44 %).

**EXEMPLE 6 : Acide 6-amino 2-éthyl 2-[(méthoxycarbonyl) amino] 4-méthylène heptanedioïque**

On introduit à 0°C 2,25 $cm^3$ de soude 2N dans une solution renfermant 225 mg de produit obtenu au stade A de l'exemple 5, dans 2,25 $cm^3$ d'éthanol. On laisse revenir à température ambiante, maintient la solution obtenue sous agitation pendant 16 heures. On dilue à l'eau et ajoute de la résine Dowex 50 W x 8. On agite 30 minutes, filtre et rince à l'eau. On élue avec de l'ammoniaque au 1/10, puis évapore à sec. On obtient 180 mg de produit que l'on purifie par chromatographie sur silice en éluant par le mélange éthanol ammoniaque (8-2). On obtient ainsi le produit recherché. Rf = 0,6.

**EXEMPLE 7 : Acide 2,6-diamino 2-éthyl 4-méthylène heptanedioïque**

On dissout 300 mg de produit obtenu au stade A de l'exemple 5 dans 3 $cm^3$ d'éthanol. On ajoute à la solution ainsi obtenue 3 $cm^3$ de lessive de soude. On maintient à 90°C pendant 13 heures. On ajoute de la glace et acidifie à pH 2 par addition de résine Dowex 50 W x 8. On filtre. On rince à l'eau. On élue avec de l'ammoniaque diluée et évapore à sec. On obtient 230 mg de produit que l'on purifie par chromatographie sur silice en éluant par le mélange éthanol-ammoniaque (8-2). On obtient 125 mg de produit recherché. Rf = 0,3 (éluant : butanol-acide acétique-eau (4-2-2).

**EXEMPLE 8 : Acide 2,6-diamino 2-(difluorométhyl) 4-méthylène heptanedioïque**

STADE A : 6-azido 2 difluorométhyle 2-formyl amino 4-méthylène heptanedioate de diéthyle.

On ajoute 0,131 g d'azoture de sodium dans une solution renfermant 0,700 g de 2-(difluorométhyl) 2-(formylamino) 4-méthylène 6-[(méthanesulfonyl) oxy] heptanedioate de diéthyle dans 15 $cm^3$ de diméthylformamide. On agite la solution obtenue pendant 16 heures à la température ambiante. On évapore le diméthylformamide à 35°C sous pression réduite. On reprend le résidu dans le chlorure de méthylène et lave avec une solution aqueuse à 10 % de carbonate acide de sodium, puis avec de la saumure, on sèche et évapore à sec. On obtient 655 mg de produit recherché. Rf = 0,65 (éluant : $CHCl_2$-AcOEt (8-2)).

STADE B : 6-amino 2-difluorométhyle 2-formyl amino 4-méthylène heptanedioate de diéthyle.

On ajoute à 0°C, 310 mg de triphénylphosphine dans une solution renfermant 350 mg de produit préparé au stade précédent et 20 $cm^3$ de tétrahydrofuranne. On laisse revenir à tem pérature ambiante et maintient la solution obtenue sous agitation pendant 16 heures. On ajoute 0,5 $cm^3$ d'eau et maintient le mélange réactionnel sous agitation pendant 24 heures.

On évapore le tétrahydrofuranne et reprend le résidu dans le chlorure de méthylène. On extrait avec une solution d'acide chlorhydrique 2N. On neutralise à l'aide d'une solution de carbonate acide de sodium, et extrait

à l'acétate d'éthyle. On lave, sèche et évapore à sec. On obtient 250 mg de produit recherché. Rf = 0,15.

STADE C : Acide 2,6-diamino 2-(difluorométhyl) 4-méthylène heptanedioïque.

a) Déformylation.

On porte au reflux pendant une heure une solution renfermant 140 mg de produit préparé au stade précédent dans 5 $cm^3$ d'éthanol et 0,5 $cm^3$ d'acide chlorhydrique 12N. On neutralise avec du carbonate acide de sodium, évapore à sec et reprend à l'eau. On extrait à l'acétate d'éthyle, lave à l'eau, sèche et évapore à sec. On obtient 100 mg de produit.

b) Saponification.

On reprend le produit obtenu au stade a) dans 3 $cm^3$ d'éthanol, et ajoute 1,5 $cm^3$ d'une solution de soude N. On maintient le mélange réactionnel sous agitation pendant 16 heures à la température ambiante. On neutralise à l'aide d'une solution d'acide chlorhydrique jusqu'à pH -~ 5. On évapore à sec, reprend le résidu dans l'eau. On dépose le produit sur une résine échangeuse d'ions (Dowex 50 W x 8). On élue à l'eau, puis avec une solution d'ammoniaque 0,7 N. On réunit et amène à sec les fractions contenant le produit recherché. On obtient ainsi 80 mg de produit recherché que l'on lyophilise.
Rf = 0,25 dans BuOH, AcOH, $H_2O$ (4-2-2).

PREPARATION 2 : 2-(difluorométhyl) 2-formylamino 4-méthylène 6-[(méthylsulfonyl) oxy] heptanedioate de diéthyle.

Stade A : (2-méthyl 2-propényl) propanedioate de (1) (1,1-diméthyléthyle) et de (3) éthyle.

On dissout 33 g de malonate d'éthyle et de terbutyle dans 400 cm3 d'acétonitrile, ajoute en agitant 29 g de carbonate de potassium, 0,5 g d'éther couronne (18-6) et 300 g de 3-chloro 2-méthyl 1-propène puis agite 16 heures à 65°C. On filtre l'insoluble et évapore à sec le filtrat. On chromatographie le résidu sur silice, élue par un mélange cyclohexane-acétate d'éthyle (95-5) et isole 18 g de produit pur et 22 g de mélange. On chromatographie celui-ci sur silice élue par un mélange cyclohexane-acétate d'éthyle (97,5-2,5) et isole encore 11 g de produit.
Rf. = 0,35 dans cyclohexane-acétate d'éthyle (95-5).

Stade B : (Difluorométhyl) (2-méthyl 2-propényl) propane dioate de (1) (1,1-diméthyléthyl) et de (3) éthyle.

On met en suspension 2,8 g d'hydrure de sodium dans 100 cm3 de tétrahydrofuranne, ajoute goutte à goutte 11,1 g de produit obtenu au stade A dans 100 cm3 de tétrahydrofuranne et agite une heure à 42°C. On fait barboter du Fréon 22 pendant 15 minutes en agitant à 45°C et agite encore une heure à 45°C et une heure à température ambiante sous atmosphère de Fréon 22. On hydrolyse le milieu réactionnel avec de l'eau salée, extrait au chlorure de méthylène, lave la phase organique à l'eau salée, sèche et concentre à sec. On obtient 13 g de produit attendu.
Rf. = 0,4 dans cyclohexane-acétate d'éthyle (95-5).

Stade C : (Difluorométhyl) (2-méthyl 2-propényl) propane dioate de monoéthyle.

On dissout 13 g de produit obtenu ci-dessus dans 100 $cm^3$ de chlorure de méthylène, ajoute 70 cm3 d'acide trifluoroacétique et agite une heure 30 minutes à température ambiante. On concentre à sec sous pression réduite, reprend le résidu par du chlorure de méthylène et extrait avec une solution aqueuse à 10 % de bicarbonate de sodium. On lave la phase aqueuse avec du chlorure de méthylène et neutralise avec de l'acide chlorhydrique concentré. On extrait au chlorure de méthylène, lave à l'eau salée, sèche et concentre à sec et obtient 5,3 g de produit attendu.
Rf. = 0,5 dans $CH_2Cl_2$ - MeOH - AcOH (9-0,5-0,5).

Stade D : 2-(difluorométhyl) 2-(formylamino) 4-méthyl 4-penténoate d'éthyle.

On agite 10,5 g de produit obtenu ci-dessus dans 60 cm3 de chlorure de thionyle et chauffe au reflux pendant 3 heures. On évapore à sec, reprend le résidu dans le toluène et sèche sous pression réduite et obtient

10,5 g de chlorure d'acide. On dissout ce dernier dans 50 cm3 d'acétone, refroidit à 0°C, ajoute goutte à goutte, une solution de 3,35 g d'azoture de sodium dans 20 cm3 d'eau et agite pendant une heure à 0°C. On évapore l'acétone, extrait à l'éther, lave à l'eau salée, sèche, évapore à sec et obtient 10 g d'azoture. On dissout ce dernier dans 100 cm3 d'acide formique et chauffe au reflux pendant une heure trente minutes. On laisse revenir à température ambiante et ajoute goutte à goutte 40 cm3 d'anhydride acétique et agite 3 heures à température ambiante. On ajoute lentement 40 cm3 d'eau glacée, évapore à sec et reprend dans un mélange eau - chlorure de méthylène, extrait au chlorure de méthylène, lave avec une solution aqueuse à 10% de bicarbonate de sodium, puis à l'eau salée, sèche et concentre à sec. On chromatographie le résidu sur silice, élue par un mélange cyclohexane-acétate d'éthyle (85-15) et isole 5 g de produit attendu.

Stade E : 2-(fluorométhyl) 2-(formylamino) 6-hydroxy 4-méthylène heptanedioate de diéthyle.

On met en suspension 9,65 g de chlorure ferrique dans 50 cm3 de chlorure de méthylène, ajoute goutte à goutte 3,03 g de glyoxylate d'éthyle dans 50 cm3 de chlorure de méthylène et agite 30 minutes à température ambiante. On refroidit à -60°C et ajoute goutte à goutte 3,5 g de produit obtenu en D dans 50 cm3 de chlorure de méthylène. On agite une heure à -30°C puis une heure à -20°C. On verse le mélange réactionnel sur de l'eau glacée, extrait au chlorure de méthylène, lave à l'eau salée puis avec une solution aqueuse à 1O% de bicarbonate de sodium puis à l'eau salée. On sèche et évapore à sec. On chromatographie sur silice le résidu, élue par un mélange cyclohexane-acétate d'éthyle (6-4) et isole 0,590 g de produit B (4-méthyl heptène...) et 1,7 g de produit C (4-méthylène -heptane...) et 1 g de mélange.
Produit B : Rf. 0,32 dans cyclohexane - acétate d'éthyle
Produit C : Rf. 0,35 (1 - 1)

Stade F : 2-(difluorométhyl) 2-(formylamino) 4-méthylène 6-[(méthylsulfonyl) oxy] heptanedioate de diéthyle.

On dissout 1,65 g de produit C obtenu ci-dessus dans 20 cm3 de pyridine, refroidit à 0°C et ajoute 0,725 g de chlorure de méthanesulfonyle. On agite 30 minutes à 0°C et 5 heures à température ambiante. On verse le mélange réactionnel sur de l'acide chlorhydrique 6N glacé, extrait au chlorure de méthylène, lave la phase organique avec de l'acide chlorhydrique 6N puis avec une solution aqueuse à 10 % de bicarbonate de sodium enfin à l'eau salée. On sèche et évapore à sec. On chromatographie le résidu sur silice, élue par un mélange chlorure de méthylène -acétate d'éthyle (9-1) et isole 1,55 g de produit attendu.
Rf. = 0,45 dans $CH_2Cl_2$ - AcOEt (8-2).

**EXEMPLE 9 : Acide 2-éthynyl 2-[(méthoxycarbonyl) amino] 4-méthylène 6-[(phénylméthyl) amino] heptanedioïque**

STADE A : 2-[(méthoxycarbonyl) amino] 4-méthylène 6-[(phénylméthyl) amino] 2-[2-(triméthylsilyl) éthynyl] heptanedioate de 7-méthyle 1-méthyle.

On ajoute une solution renfermant 1,6 g de produit préparé à la préparation 3 dans 40 $cm^3$ de chlorure de méthylène, dans une solution renfermant 387 mg de triéthylamine, 393 mg de benzylamine dans 80 $cm^3$ de chlorure de méthylène. On agite 6 heures à la température ambiante. On lave à l'aide d'une solution d'acide chlorhydrique N, puis à l'aide d'une solution de bicarbonate de sodium. On sèche et évapore à sec. On obtient 1,54 g de produit recherché brut que l'on purifie par chromatographie sur silice en éluant par le mélange chlorure de méthylène, acétate d'éthyle (95-5). On obtient 1,2 g de produit recherché.
Rf = 0,28 ($CH_2Cl_2$ AcOEt (9-1)).

STADE B : 2-éthynyl 2-[(méthoxycarbonyl) amino] 4-méthylène 6-[(phénylméthyl) amino] heptanedioate de 7-éthyle 1-méthyle.

On ajoute 0,23 g de fluorure de potassium à 1,23 g de produit préparé comme au stade A, en solution dans 30 $cm^3$ de diméthylformamide. On agite 16 heures à la température ambiante. On dilue dans 200 $cm^3$ d'éther éthylique. On lave, sèche et évapore à sec. On obtient 1,02 g de produit recherché. Rf = 0,6 (cyclohexane-acétate d'éthyle (7-3)).

STADE C : Acide 2-éthynyl 2-[(méthoxycarbonyl) amino] 4-méthylène 6-[(phénylméthyl) amino] heptanedioïque.

On ajoute 0,5 $cm^3$ de soude 2N à une solution renfermant 140 mg de produit préparé au stade précédent et 15 $cm^3$ d'éthanol. On agie à la température ambiante pendant 3 jours. On neutralise avec de la résine Dowex 50 W x 8. On filtre, lave à l'eau puis élue avec 100 $cm^3$ d'ammoniaque 0,5N. On évapore à sec, reprend le résidu à l'eau, filtre et lyophilise. On obtient 106 mg de produit recherché.
Rf = 0,25 (EtOH, $NH_4OH$ (9-1)).

PREPARATION 3 : 2-[(méthoxycarbonyl) amino] 4-méthylène 6-[(trifluorométhylsulfonyl) oxy] 2-[2-(triméthylsilyl) éthynyl] heptanedioate de 7-éthyle 1-méthyle.

On ajoute à 0°C 1,015 g d'anhydride trifluorométhane sulfonique dans une solution renfermant 594 mg de pyridine et 6 $cm^3$ de chlorure de méthylène. On agite la solution obtenue pendant 10 minutes à 0°C. On verse la solution obtenue dans une solution renfermant 1,2 g de produit préparé au stade B de la préparation 1 en solution dans 30 $cm^3$ de chlorure de méthylène. On agite 15 minutes à 0°C. On lave à l'acide chlorhydrique N, puis avec une solution de carbonate acide de sodium. On sèche et évapore à sec à 30°C. On obtient 1,6 g de produit recherché.
Rf = 0,45 (cyclohexane-acétate d'éthyle (7-3)).

**EXEMPLE 10 : Acide 2-amino 2-éthynyl 4-méthylène 6-[(phénylméthyl) amino] heptanedioïque**

On chauffe pendant 2 h 30 à 90°C, une solution renfermant 400 mg de produit préparé à l'exemple 9, dans 35 $cm^3$ de soude 6N. On neutralise avec de l'acide chlorhydrique 12N jusqu'à l'obtention d'un pH voisin de 5. On concentre partiellement. On dépose sur de la résine Dowex 50 W x 8. On rince à l'eau, élue à l'ammoniaque 0,5N puis réunit les fractions contenant le produit. On évapore à sec et obtient 320 mg de produit que l'on purifie sur silice en éluant avec le mélange chlorure de méthylène-méthanol-acide acétique (5-4-1). On obtient 150 mg de produit que l'on purifie de nouveau par chromatographie sur silice en éluant par le mélange $CH_2Cl_2$ - MeOH - $H_2O$ (5-5-2) puis par le mélange $CH_2Cl_2$ - MeOH - $NH_4OH$ (5-5-1). On obtient ainsi le produit recherché pur que l'on lyophilise.
Rf = 0,35 (éluant : BeOH - AcOH - $H_2O$ (4-2-2)).

**EXEMPLE 11 : Acide 2-amino 6-(diméthylamino) 2-éthynyl 4-méthylène heptanedioïque.**

STADE A : 2-[(méthoxycarbonyl) amino] 6-(diméthylamino) 4-méthylène 2-(2-triméthylsilyl) éthynyl] heptanedioate de 7-éthyl 1-méthyle.

On refroidit à 0°C 1,44 g de 2-[(méthoxycarbonyl) amino] 4-méthylène 6-[(trifluorométhylsulfonyl) oxy] 2-[2-(triméthylsilyl) éthynyl heptanedioate de 7-éthyl 1-méthyle préparé comme à la préparation 3 dans 40 $cm^3$ de tétrahydrofuranne. On fait barboter pendant 5 minutes de la diméthylamine, puis agite 30 minutes à 0°C. On évapore le solvant, reprend dans du chlorure de méthylène, lave avec une solution aqueuse à 10% de bicarbonate de soude, concentre à sec, récupère 1,2 g de produit brut que l'on purifie par chromatographie sur silice (éluant : chlorure de méthylène-acétate d'éthyle 6-4). On obtient 440 mg de produit attendu.
Rf = 0,33 (chlorure de méthylène-acétate d'éthyle 6-4).

STADE B: Acide 2-amino 6-(diméthylamino) 2-éthynyl 4-méthylène heptanedioïque.

On ajoute 108 mg de fluorure de potassium dans 400 mg de produit obtenu au stade A en solution dans 15 $cm^3$ de diméthylformamide et agite 16 heures à température ambiante. On concentre à sec, reprend au chlorure de méthylène, lave à l'eau salée, sèche et évapore les solvants. On obtient 330 mg d'intermédiaire 2-éthynyl. On dissout 300 mg de ce produit dans 10 $cm^3$ d'éthanol, ajoute 1,7 $cm^3$ de soude 2N puis agite 16 heures à température ambiante. On évapore le solvant, reprend le résidu dans 4 $cm^3$ de soude 6N et agite 4 heures à 90°C. On neutralise par traitement sur résine Dowex pendant 1 heure. On filtre, lave à l'eau, puis avec de l'ammoniaque 0,5N. On obtient 270 mg de produit brut que l'on chromatographie sur silice (éluant : éthanol-ammoniaque 9-1, 8-2 puis 7-3). On traite de nouveau sur résine Dowex, élue à l'ammoniaque 0,5N, reprend dans 10 $cm^3$ d'eau et lyophilise. On obtient 135 mg de produit attendu.
Rf = 0,25 (éthanol-ammoniaque 8-2).

**EXEMPLE 12 : Acide 2-éthynyl 2-[(méthoxycarbonyl) amino] 4-méthylène 6-(méthylamino) heptanedioïque.**

STADE A : 2-[(méthoxycarbonyl) amino] 6-(méthylamino) 4-méthylène 2-[2-(triméthylsilyl) éthynyl] heptanedioate de 7-éthyl 1-méthyle.

On ajoute 1,4 $cm^3$ de méthylamine en solution éthanolique (8,06 M/l) dans une solution comprenant 0,96 g de 2-[(méthoxycarbonyl) amino] 4-méthylène 6-[(trifluorométhylsulfonyl) oxy] 2-[2-(triméthylsilyl) éthynyl] heptanedioate de 7-éthyl 1-méthyle préparé comme à la préparation 3 dans 25 $cm^3$ de tétrahydrofuranne puis agite 15 minutes à température ambiante. On évapore le solvant, reprend le résidu dans du chlorure de méthylène, lave avec une solution aqueuse à 10% de bicarbonate de sodium, sèche et concentre à sec. On obtient 770 mg de produit que l'on chromatographie sur silice (éluant : chlorure de méthylène-acétate d'éthyle 6-4). On recueille 530 mg de produit attendu.
Rf = 0,15 (chlorure de méthylène-acétate d'éthyle 6-4).

STADE B : 2-éthynyl 2-[(méthoxycarbonyl) amino] 6-(méthylamino) 4-méthylène heptanedioate de 7-éthyl 1-méthyle.

On opère comme au stade B de l'exemple 9 en utilisant 850 mg de produit préparé comme au stade A dans 30 $cm^3$ de diméthylformamide et 260 mg de fluorure de potassium. On obtient 620 mg de produit attendu.
Rf = 0,5 (chlorure de méthylène-acétate d'éthyle 5-5).

STADE C : Acide 2-éthynyl 2-[(méthoxycarbonyl) amino] 4-méthylène 6-(méthylamino) heptanedioïque.

On opère comme au stade C de l'exemple 9 en utilisant au départ 200 mg de produit obtenu au stade B dans 15 $cm^3$ d'éthanol et 0,9 $cm^3$ de soude 2N. On obtient 156 mg de produit attendu.
Rf = 0,3 (éthanol-ammoniaque 8-2).

**EXEMPLE 13 : Acide 2-amino 6-(méthylamino) 2-éthynyl 4-méthylène heptanedioïque.**

On chauffe 3 heures à 90°C une solution renfermant 320 mg de produit obtenu comme à l'exemple 12 dans 35 $cm^3$ de soude 6N. On lave avec de l'acide chlorhydrique 12N jusqu'à pH = 3. On traite sur résine Dowex 50 W x 8, rince à l'eau, élue à l'ammoniaque 0,5N puis réunit les fractions contenant le produit. On recueille 220 mg de produit que l'on purifie sur silice (éluant : éthanol-ammoniaque 95-5, 90-10 puis 85-15). Après un nouveau traitement sur résine Amberlyst 15, on lave à l'eau et élue à l'ammoniaque 0,5N, lyophilise et obtient 53 mg de produit pur attendu.
Rf = 0,2 (éthanol-ammoniaque 85-15).

**EXEMPLE 14 :**

On a préparé des comprimés répondant à la formule
- Produit de l'exemple 2 50 mg
- Excipient q. s. pour un comprimé terminé à 250 mg

(détail de l'excipient : lactose, amidon, talc, stéarate de magnésium).

**EXEMPLE 15 :**

On a préparé des gélules répondant à la formule
- Produit de l'exemple 3 100 mg
- Excipient classique pour des gélules.

Activité antibactérienne (in vitro)

L'activité antibactérienne des produits revendiqués a été déterminée par la méthode de diffusion en milieu de Davis Mingioli additionné de 1% d'agar. Les géloses utilisées sont coulées en boîtes de Petri à 48°C, après ensemencement à $5x10^{-5}$ germes/ml au moyen de la souche bactérienne test. Les inocula proviennent d'une pré-culture de 24 heures en bouillon de Davis Mingioli. Après durcissement des géloses, les solutions aqueuses des produits étudiés sont introduites dans des puits (9 mm) creusés dans le milieu à l'aide d'un emporte-pièce. Les zones d'inhibition observées (diamètre en mm) sont mesurées après incubation de 24 heures à 37°C.

| | Produit de l'exemple 4 (100 mg/l) | Produit de l'exemple 7 (100 mg/l) | Produit de l'exemple 10 (100 mg/l) |
|---|---|---|---|
| Escherichia Coli 078 | 17,5 | 18 | |
| Salmonella typhimurium MZ11 | 31 | 34 | 19 |
| Enterobacter cloacae 1321E | 26 | 29 | |
| Providencia sp. DU48 | 29 | 28 | |

## Revendications

### Revendications pour les Etats contractants suivants : AT, BE, CH, DE, DK, FR, GB, IT, LI, LU, NL, SE

**1.** Les composés de formule générale (I) :

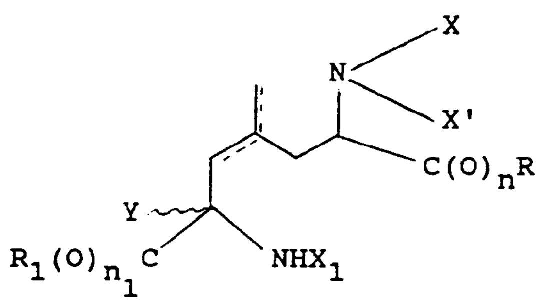

(I)

dans lesquels,

- les traits pointillés représentent une double liaison éventuelle endo ou exo,
- Y représente un radical alkyle, alcényle ou alcynyle renfermant de 2 à 18 atomes de carbone, ou un radical alkyle renfermant de 1 à 18 atomes de carbone substitué par un ou plusieurs atomes d'halogène,
- X, X' et $X_1$ identiques ou différents représentent un atome d'hydrogène, un radical alkyle, alcényle ou alcynyle renfermant jusqu'à 8 atomes de carbone, un radical aryle renfermant jusqu'à 14 atomes de carbone, un radical aralkyle renfermant jusqu'à 18 atomes de carbone, ou un radical acyle dérivé d'un acide gras et/ou d'un acide aminé alpha et/ou oméga,

et soit n et/ou $n_1$ représente le nombre 1 et R et/ou $R_1$ représente le reste d'une amine ou d'un acide aminé alpha ou oméga,

soit n et/ou $n_1$ représente le nombre 2 et R et $R_1$, représentent un atome d'hydrogène, un radical alkyle renfermant jusqu'à 8 atomes de carbone ou un radical aryle renfermant jusqu'à 14 atomes de carbone, ou un radical aralkyle renfermant jusqu'à 18 atomes de carbone, ou un radical

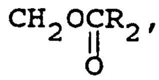

dans lesquels $R_2$ représente un radical alkyle renfermant jusqu'à 8 atomes de carbone, ou un radical aryle renfermant jusqu'à 14 atomes de carbone, ainsi que leurs sels avec les acides organiques ou minéraux ou avec les bases.

2. Les composés de formule (I) tels que définis à la revendication 1, dans lesquels les traits pointillés représentent une double liaison exo ainsi que leurs sels avec les acides organiques ou minéraux ou avec les bases.

3. Les composés de formule (I) tels que définis à la revendication 1 ou 2, dans lesquels X′ représente un atome d'hydrogène, ainsi que leurs sels avec les acides organiques ou minéraux ou avec les bases.

4. Les composés de formule (I) tels que définis à l'une quelconque des revendications 1 à 3, dans lesquels X, X′ et $X_1$ représentent chacun un atome d'hydrogène ainsi que leurs sels avec les acides organiques ou minéraux, ou avec les bases.

5. Les composés de formule (I) tels que définis à la revendication 1 ou 2, dans lesquels X′ représente un atome d'hydrogène ou un radical méthyle, X représente un radical méthyle ou benzyle et $X_1$ représente un atome d'hydrogène ainsi que leurs sels avec les acides organiques ou minéraux, ou avec les bases.

6. Les composés de formule (I) tels que définis à l'une quelconque des revendications 1 à 5, dans lesquels R et $R_1$ représentent chacun un atome d'hydrogène ainsi que leurs sels avec les acides organiques ou minéraux, ou avec les bases.

7. Les composés de formule (I) tels que définis à l'une quelconque des revendications 1 à 6, dans lesquels Y représente un radical acétylénique ainsi que leurs sels avec les acides organiques ou minéraux ou avec les bases.

8. Les composés de formule (I) tels que définis à l'une quelconque des revendications 1 à 6, dans lesquels Y représente un radical éthylénique ainsi que leurs sels d'addition avec les acides organiques ou minéraux, ou avec les bases.

9. Les composés de formule (I) tels que définis à l'une quelconque des revendications 1 à 6, dans lesquels Y représente un radical éthyle ainsi que leurs sels avec les acides organiques ou minéraux, ou avec les bases.

10. Les composés de formule (I) tels que définis à la revendication 1, dont les noms suivent :
    - l'acide 2,6-diamino 2-éthényl 4-méthylène heptanedioïque,
    - l'acide 2,6-diamino 2-éthyl 4-méthylène heptanedioïque,
    - l'acide 2-amino 2-éthynyl 4-méthylène 6-(phénylméthyl) amino heptanedioïque.

11. A titre de médicaments, les composés de formule (I) tels que définis à l'une quelconque des revendications 1 à 9, ainsi que leurs sels avec les acides organiques ou minéraux ou avec les bases pharmaceutiquement acceptables.

12. A titre de médicaments, les composés de formule (I) tels que définis à la revendication 10, ainsi que leurs sels avec les acides organiques ou minéraux ou avec les bases pharmaceutiquement acceptables.

13. Procédé de préparation des composés de formule (I) tels que définis à l'une quelconque des revendications 1 à 10, caractérisé en ce que l'on soumet un composé de formule générale (II) :

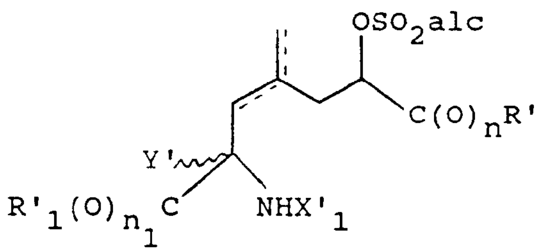

(II)

dans laquelle alc représente un radical alkyle renfermant de 1 à 8 atomes de carbone eventuellement substitué par un ou plusieurs atome d'halogène, n, $n_1$ et les traits pointillés conservent la même signification que précédemment, Y' représente soit Y, soit un précurseur de Y, $X'_1$, R' et R'1 ont la même signification que $X_1$, R et $R_1$ à l'exception de l'hydrogène, à l'action d'un réactif capable de fournir un précurseur de la fonction amino ou d'une amine de formule

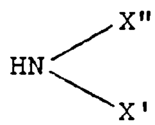

dans laquelle X' a la signification indiquée à la revendication 1 et X" a la signification indiquée pour X à l'exception de l'hydrogène pour obtenir le cas échéant après traitement approprié le composé de formule (I) que l'on soumet, si désiré, à la totalité ou à une partie seulement des opérations suivantes dans un ordre quelconque :

- déprotection des fonctions amines,
- fonctionnalisation des fonctions amines,
- hydrolyse des fonctions esters,
- traitement de Y' pour obtenir Y,
- réduction totale ou sélective du substituant Y lorsque celui-ci est insaturé,
- salification.

**14.** Procedé selon la revendication 13, caractérisé en ce que le réactif capable de fournir un précurseur de la fonction amino est l'azoture de sodium, le phtalimide de potassium ou l'hydroxylamine.

**15.** Procédé selon la revendication 13, caractérisé en ce que l'on soumet un composé de formule (II) défini comme à la revendication 13, à l'action de l'azoture de sodium pour obtenir le composé de formule (III) :

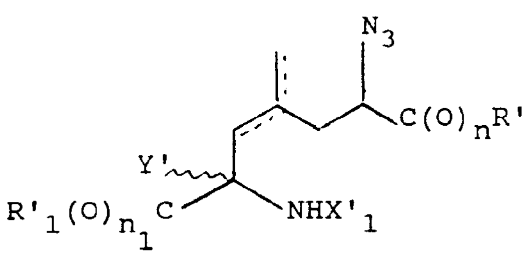

(III)

que l'on soumet à l'action d'un agent de réduction du groupement $N_3$, pour obtenir le composé de formule (IV) :

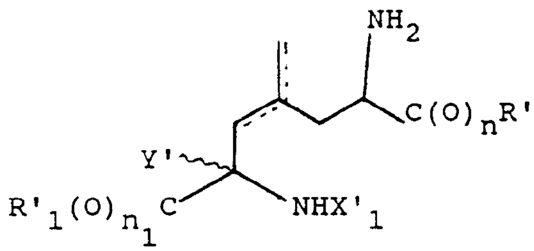

(IV)

que l'on soumet à la totalité ou à une partie seulement des opérations mentionnées à la fin de la revendication 13.

**16.** Procédé selon la revendication 13, caractérisé en ce que l'on soumet un composé de formule ($II_A$) :

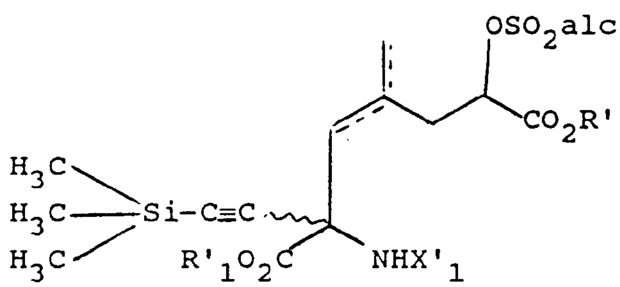

($II_A$)

soit à l'action de l'azoture de sodium pour obtenir le composé de formule ($III_A$) :

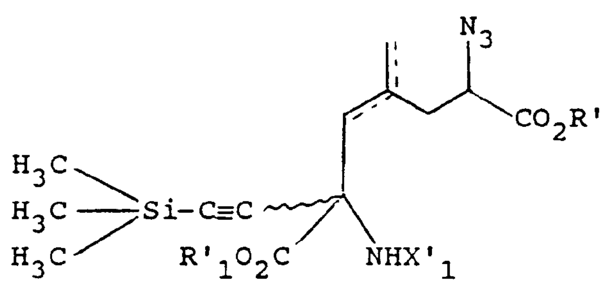

($III_A$)

que l'on soumet à l'action d'un agent de réduction de l'azoture pour obtenir le composé de formule ($IV_A$) :

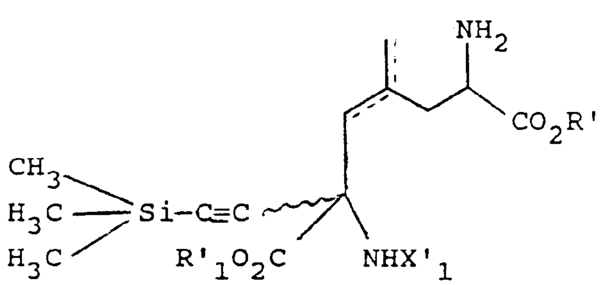

(IV)

soit à l'action d'une amine de formule

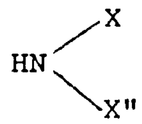

dans laquelle X' et X" ont la signification indiquée à la revendication 13 pour obtenir le composé de formule ($IV_B$) :

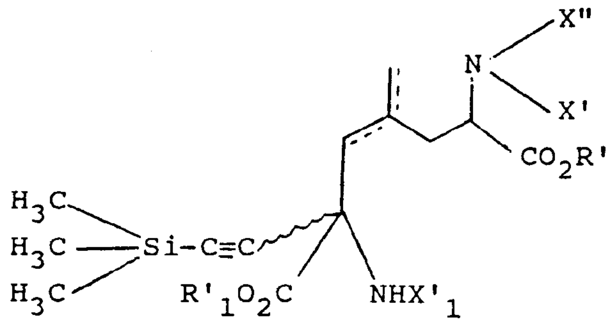

($IV_B$)

puis ou bien soumet le composé ($IV_A$) ou ($IV_B$) à l'action d'un agent de clivage sélectif des fonctions esters et du radical triméthylsilyle pour obtenir le composé de formule ($I_A$) :

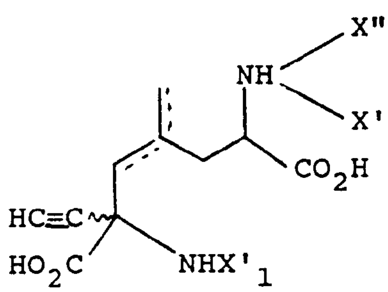

($I_A$)

que le cas échéant l'on soumet à l'action d'un agent de clivage du groupement protecteur d'amine pour obtenir le composé de formule ($I_B$) :

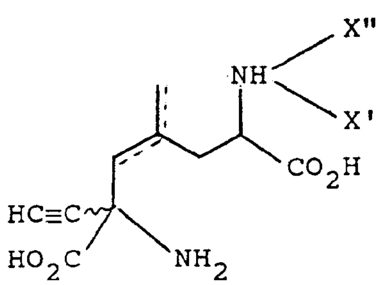

($I_B$)

ou bien soumet le composé de formule ($IV_A$) ou ($IV_B$) à l'action d'un agent de clivage du triméthylsilyle pour obtenir le composé de formule ($I_C$) :

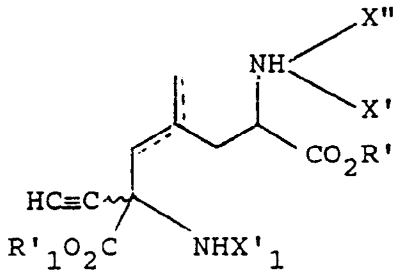

$(I_C)$

puis l'on soumet si désiré, le composé de formule $(I_A)$, $(I_B)$ ou $(I_C)$ à l'action d'un agent de réduction partielle pour obtenir le composé de formule $(I_D)$ correspondant dans lequel Y représente un radical éthényle, ou à l'action d'agent de réduction totale de la triple liaison pour obtenir le composé de formule $(I_E)$ correspondant dans lequel Y est un radical éthyle,
puis le cas échéant soumet le composé de formule (I) ainsi obtenu à la totalité ou à une partie seulement des opérations mentionnées à la fin de la revendication 13.

**17.** A titre de produits chimique nouveaux, les composés de formule (II) tels que définis à la revendication 13, dans lesquels Y' est un radical triméthylsilyle alkynyle renfermant jusqu'à 8 atomes de carbone.

**18.** A titre de produits chimiques nouveaux, les composés de formule (III) et $(III_A)$ tels que définis aux revendications 15 et 16.

**Revendications pour l'Etat contractant suivant : ES**

**1.** Procédé de préparation des composés de formule générale (I) :

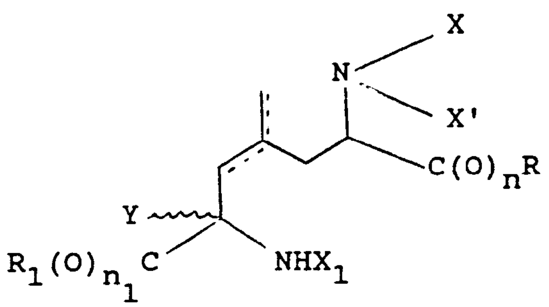

(I)

dans lesquels,
- les traits pointillés représentent une double liaison éventuelle endo ou exo,
- Y représente un radical alkyle, alcényle ou alcynyle renfermant de 2 à 18 atomes de carbone, ou un radical alkyle renfermant de 1 à 18 atomes de carbone substitué par un ou plusieurs atomes d'halogène,
- X, X' et $X_1$ identiques ou différents représentent un atome d'hydrogène, un radical alkyle, alcényle ou alcynyle renfermant jusqu'à 8 atomes de carbone, un radical aryle renfermant jusqu'à 14 atomes de carbone, un radical aralkyle renfermant jusqu'à 18 atomes de carbone, ou un radical acyle dérivé d'un acide gras et/ou d'un acide aminé alpha et/ou oméga,

et soit n et/ou $n_1$ représente le nombre 1 et R et/ou $R_1$ représente le reste d'une amine ou d'un acide aminé alpha ou oméga,
soit n et/ou $n_1$ représente le nombre 2 et R et $R_1$, représentent un atome d'hydrogène, un radical alkyle renfermant jusqu'à 8 atomes de carbone ou un radical aryle renfermant jusqu'à 14 atomes de carbone, ou un radical aralkyle renfermant jusqu'à 18 atomes de carbone, ou un radical

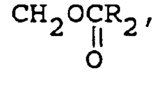

dans lesquels $R_2$ représente un radical alkyle renfermant jusqu'à 8 atomes de carbone, ou un radical aryle

renfermant jusqu'à 14 atomes de carbone, ainsi que leurs sels avec les acides organiques ou minéraux ou avec les bases, caractérisé en ce que l'on soumet un composé de formule générale (II) :

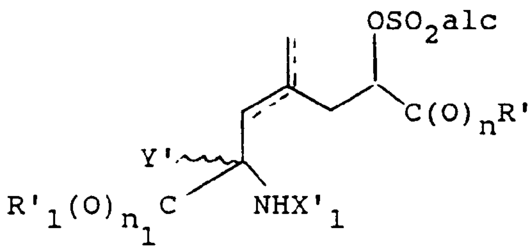

(II)

dans laquelle alc représente un radical alkyle renfermant de 1 à 8 atomes de carbone éventuellement substitué par un ou plusieurs atome d'halogène, n, $n_1$ et les traits pointillés conservent la même signification que précédemment, Y' représente soit Y, soit un précurseur de Y, $X'_1$, R' et R'1 ont la même signification que $X_1$, R et $R_1$ à l'exception de l'hydrogène, à l'action d'un réactif capable de fournir un précurseur de la fonction amino ou d'une amine de formule

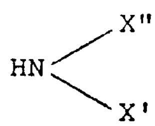

dans laquelle X' a la signification indiquée à la revendication 1 et X" a la signification indiquée pour X à l'exception de l'hydrogène pour obtenir le cas échéant après traitement approprié le composé de formule (I) que l'on soumet, si désiré, à la totalité ou à une partie seulement des opérations suivantes dans un ordre quelconque :

- déprotection des fonctions amines,
- fonctionnalisation des fonctions amines,
- hydrolyse des fonctions esters,
- traitement de Y' pour obtenir Y,
- réduction totale ou sélective du substituant Y lorsque celui-ci est insaturé,
- salification.

2. Procédé selon la revendication 1, pour la préparation des produits de formule (I) telle que définie à la revendication 1, répondant à la formule (I') :

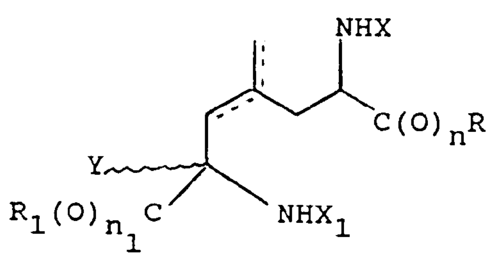

(I')

dans laquelle X, $X_1$, R, $R_1$, n, $n_1$ et Y ont la signification indiquée précédemment, caractérisé en ce que l'on fait agir sur le composé de formule (II), un réactif capable de fournir un précurseur de la fonction amino ou une amine de formule

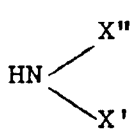

dans laquelle X″ a la signification indiquée précédemment et X′ représente un atome d'hydrogène.

**3.** Procédé selon la revendication 2, caractérisé en ce que le réactif capable de fournir un précurseur de la fonction amino est l'azoture de sodium, le phtalimide de potassium ou l'hydroxylamine.

**4.** Procédé selon la revendication 2, caractérisé en ce que l'on soumet un composé de formule (II) défini comme à la revendication 13, à l'action de l'azoture de sodium pour obtenir le composé de formule (III) :

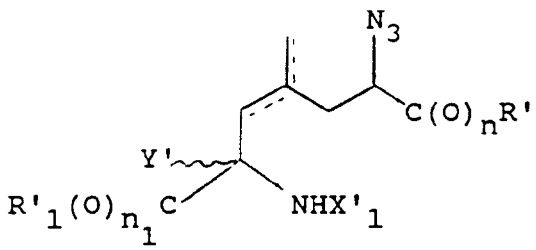

(III)

que l'on soumet à l'action d'un agent de réduction du groupement $N_3$, pour obtenir le composé de formule (IV) :

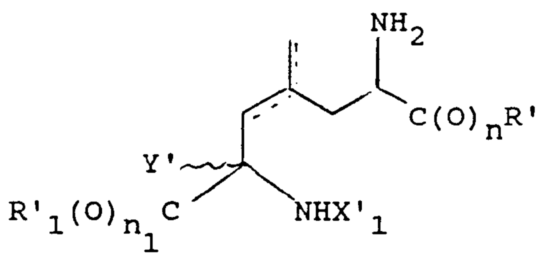

(IV)

que l'on soumet à la totalité ou à une partie seulement des opérations mentionnées à la fin de la revendication 1.

**5.** Procédé selon la revendication 1, caractérisé en ce que l'on soumet un composé de formule ($II_A$) :

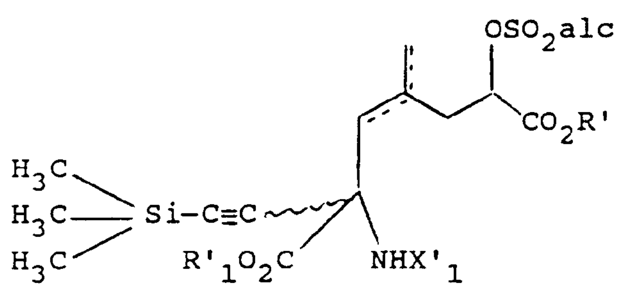

($II_A$)

<u>soit</u> à l'action de l'azoture de sodium pour obtenir le composé de formule ($III_A$) :

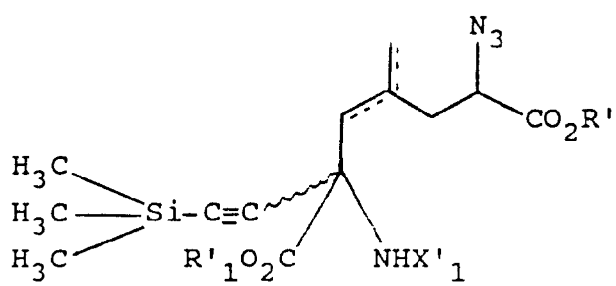

($III_A$)

que l'on soumet à l'action d'un agent de réduction de l'azoture pour obtenir le composé de formule ($IV_A$) :

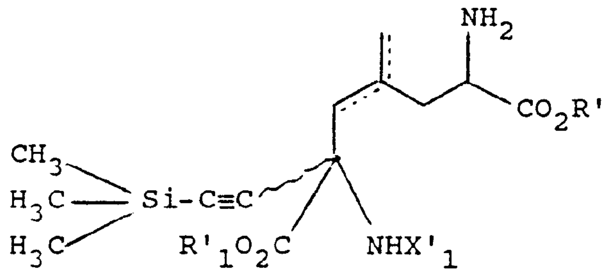

($IV_A$)

soit à l'action d'une amine de formule

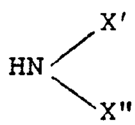

dans laquelle X′ et X″ ont la signification indiquée à la revendication 13 pour obtenir le composé de formule ($IV_B$) :

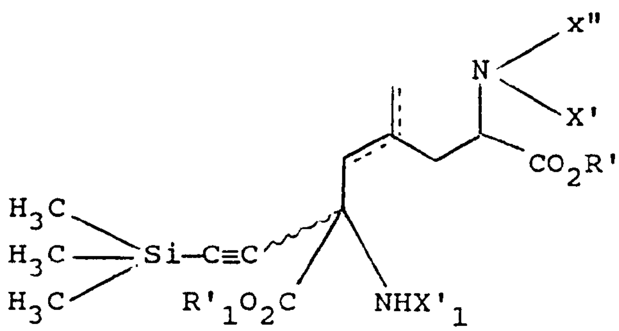

($IV_B$)

puis ou bien soumet le composé ($IV_A$) ou ($IV_B$) à l'action d'un agent de clivage sélectif des fonctions esters et du radical triméthylsilyle pour obtenir le composé de formule ($I_A$) :

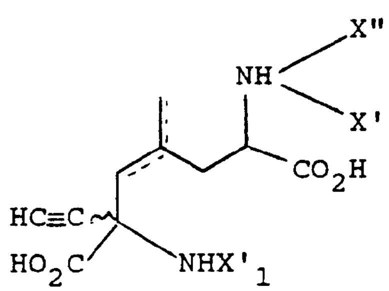

($I_A$)

que le cas échéant l'on soumet à l'action d'un agent de clivage du groupement protecteur d'amine pour obtenir le composé de formule ($I_B$) :

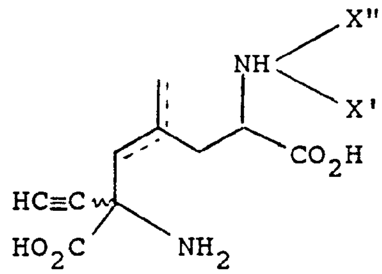

($I_B$)

ou bien soumet le composé de formule ($IV_A$) ou ($IV_B$) à l'action d'un agent de clivage du triméthylsilyle pour obtenir le composé de formule ($I_C$) :

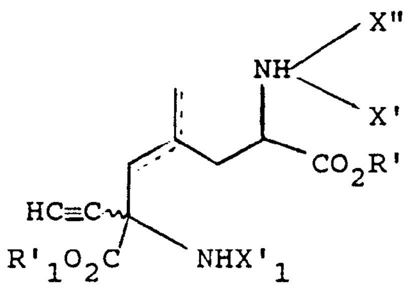

($I_C$)

puis l'on soumet si désiré, le composé de formule ($I_A$), ($I_B$) ou ($I_C$) à l'action d'un agent de réduction partielle pour obtenir le composé de formule ($I_D$) correspondant dans lequel Y représente un radical éthényle, ou à l'action d'agent de réduction totale de la triple liaison pour obtenir le composé de formule ($I_E$) correspondant dans lequel Y est un radical éthyle,
puis le cas échéant soumet le composé de formule (I) ainsi obtenu à la totalité ou à une partie seulement des opérations mentionnées à la fin de la revendication 1.

**6.** Procédé selon la revendication 5, caractérisé en ce que l'on soumet un composé de formule ($II_A$) telle que définie à la revendication 5, à l'action de l'azoture de sodium pour obtenir le composé de formule ($III_A$) telle que définie à la revendication 5, que l'on soumet à l'action d'un agent de réduction de l'azoture pour obtenir le composé de formule ($IV_A$) telle que définie à la revendication 5, puis ou bien soumet ce composé ($IV_A$) à l'action d'un agent de clivage sélectif des fonctions esters et du radical triméthylsilyle pour obtenir le composé de formule ($I'_A$) :

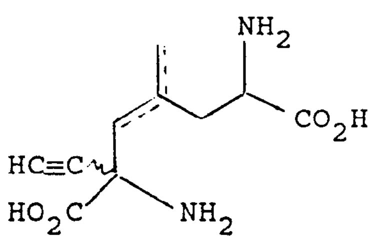

($I'_A$)

que le cas échéant l'on soumet à l'action d'un agent de clivage du groupement protecteur d'amine pour obtenir le composé de formule ($I'_B$) :

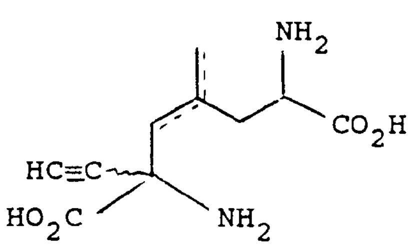

($I'_B$)

ou bien soumet ce composé de formule ($IV_A$) à l'action d'un agent de clivage du triméthylsilyle pour obtenir le composé de formule ($I'_C$) :

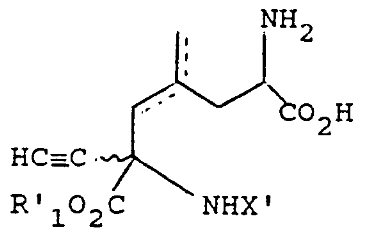

($I'_C$)

puis soumet si désiré, le composé de formule ($I'_A$), ($I'_B$) ou ($I'_C$) à l'action d'un agent de réduction partielle pour obtenir le composé de formule ($I'_D$) correspondant dans lequel Y représente un radical éthényle, ou à l'action d'agent de réduction totale de la triple liaison pour obtenir le composé de formule ($I'_E$) correspondant dans lequel Y est un radical éthyle,
puis si désiré soumet chacun des composés de formule (I) ainsi obtenus à la totalité ou à une partie seulement des opérations mentionnées à la fin de la revendication 1.

7. Procédé selon la revendication 1, caractérisé en ce que l'on utilise au départ un composé de formule (II) dans laquelle les traits pointillés représentent une double liaison exo.

8. Procédé selon la revendication 1, caractérisé en ce que l'on utilise au départ une amine de formule

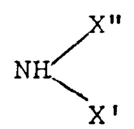

dans laquelle X′ représente un atome d'hydrogène ou un radical méthyle et X″ représente un radical méthyle ou benzyle.

9. Procédé selon la revendication 1, caractérisé en ce que l'on utilise au départ un composé de formule (II) dans laquelle Y′ représente un radical acétylénique ou un précurseur d'un radical acétylénique.

**Revendications pour l'Etat contractant suivant : GR**

1. Procédé de préparation des composés de formule générale (I) :

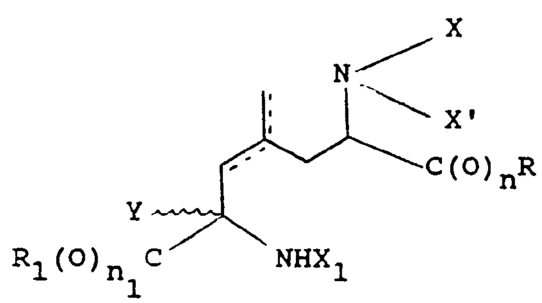

(I)

dans lesquels,
- les traits pointillés représentent une double liaison éventuelle endo ou exo,
- Y représente un radical alkyle, alcényle ou alcynyle renfermant de 2 à 18 atomes de carbone, ou un radical alkyle renfermant de 1 à 18 atomes de carbone substitué par un ou plusieurs atomes d'halogène,
- X, X′ et $X_1$ identiques ou différents représentent un atome d'hydrogène, un radical alkyle, alcényle ou alcynyle renfermant jusqu'à 8 atomes de carbone, un radical aryle renfermant jusqu'à 14 atomes

de carbone, un radical aralkyle renfermant jusqu'à 18 atomes de carbone, ou un radical acyle dérivé d'un acide gras et/ou d'un acide aminé alpha et/ou oméga,

et soit n et/ou $n_1$ représente le nombre 1 et R et/ou $R_1$ représente le reste d'une amine ou d'un acide aminé alpha ou oméga,

soit n et/ou $n_1$ représente le nombre 2 et R et $R_1$, représentent un atome d'hydrogène, un radical alkyle renfermant jusqu'à 8 atomes de carbone ou un radical aryle renfermant jusqu'à 14 atomes de carbone, ou un radical aralkyle renfermant jusqu'à 18 atomes de carbone, ou un radical

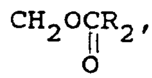

dans lesquels $R_2$ représente un radical alkyle renfermant jusqu'à 8 atomes de carbone, ou un radical aryle renfermant jusqu'à 14 atomes de carbone, ainsi que leurs sels avec les acides organiques ou minéraux ou avec les bases, caractérisé en ce que l'on soumet un composé de formule générale (II) :

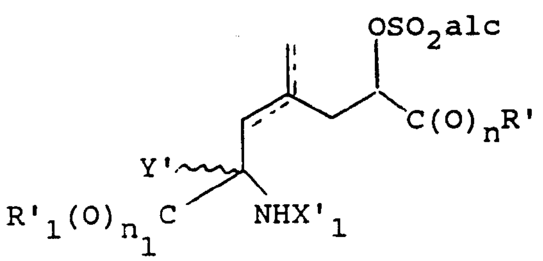

(II)

dans laquelle alc représente un radical alkyle renfermant de 1 à 8 atomes de carbone éventuellement substitué par un ou plusieurs atome d'halogène, n, $n_1$ et les traits pointillés conservent la même signification que précédemment, Y' représente soit Y, soit un précurseur de Y, $X'_1$, R' et R'1 ont la même signification que $X_1$, R et $R_1$ à l'exception de l'hydrogène, à l'action d'un réactif capable de fournir un précurseur de la fonction amino ou d'une amine de formule

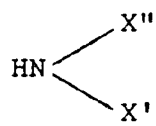

dans laquelle X' a la signification indiquée à la revendication 1 et X" a la signification indiquée pour X à l'exception de l'hydrogène pour obtenir le cas échéant après traitement approprié le composé de formule (I) que l'on soumet, si désiré, à la totalité ou à une partie seulement des opérations suivantes dans un ordre quelconque :

- déprotection des fonctions amines,
- fonctionnalisation des fonctions amines,
- hydrolyse des fonctions esters,
- traitement de Y' pour obtenir Y,
- réduction totale ou sélective du substituant Y lorsque celui-ci est insaturé,
- salification.

2. Procédé selon la revendication 1, pour la préparation des produits de formule (I) telle que définie à la revendication 1, répondant à la formule (I') :

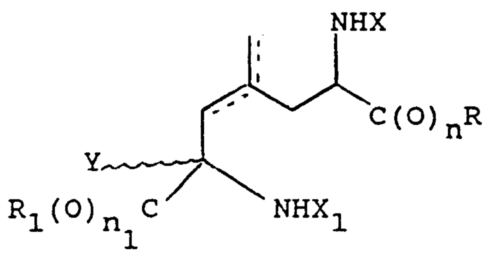

(I')

dans laquelle X, $X_1$, R, $R_1$, n, $n_1$ et Y ont la signification indiquée précédemment, caractérisé en ce que l'on fait agir sur le composé de formule (II), un réactif capable de fournir un précurseur de la fonction amino ou une amine de formule

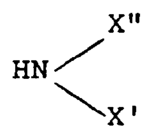

dans laquelle X" a la signification indiquée précédemment et X' représente un atome d'hydrogène.

3. Procédé selon la revendication 2, caractérisé en ce que le réactif capable de fournir un précurseur de la fonction amino est l'azoture de sodium, le phtalimide de potassium ou l'hydroxylamine.

4. Procédé selon la revendication 2, caractérisé en ce que l'on soumet un composé de formule (II) défini comme à la revendication 13, à l'action de l'azoture de sodium pour obtenir le composé de formule (III) :

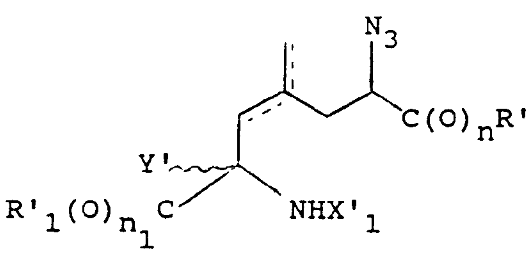

(III)

que l'on soumet à l'action d'un agent de réduction du groupement $N_3$, pour obtenir le composé de formule (IV) :

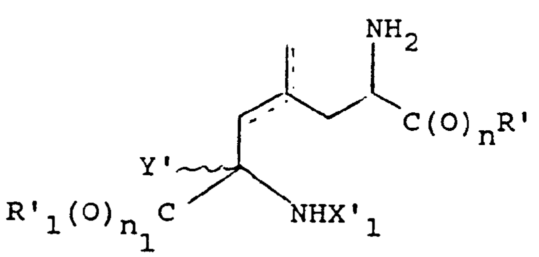

(IV)

que l'on soumet à la totalité ou à une partie seulement des opérations mentionnées à la fin de la revendication 1.

5. Procédé selon la revendication 1, caractérisé en ce que l'on soumet un composé de formule ($II_A$) :

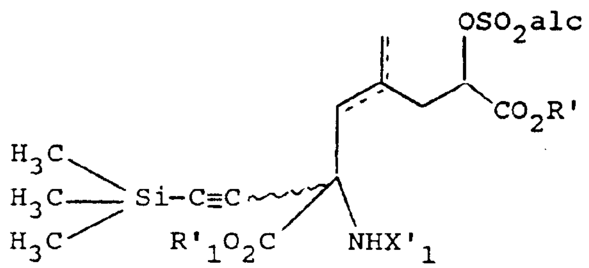

($II_A$)

soit à l'action de l'azoture de sodium pour obtenir le composé de formule ($III_A$) :

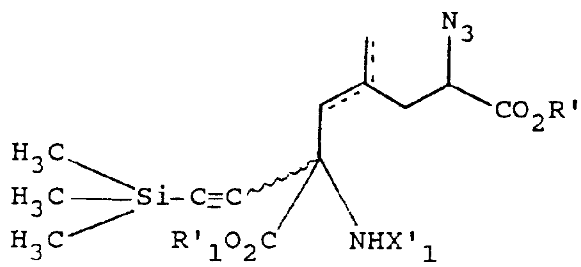

($III_A$)

que l'on soumet à l'action d'un agent de réduction de l'azoture pour obtenir le composé de formule ($IV_A$) :

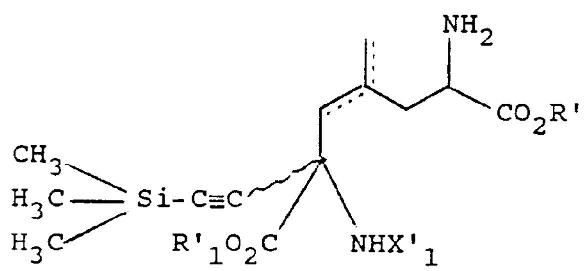

($IV_A$)

soit à l'action d'une amine de formule

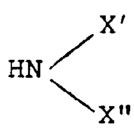

dans laquelle X' et X" ont la signification indiquée à la revendication 13 pour obtenir le composé de formule ($IV_B$) :

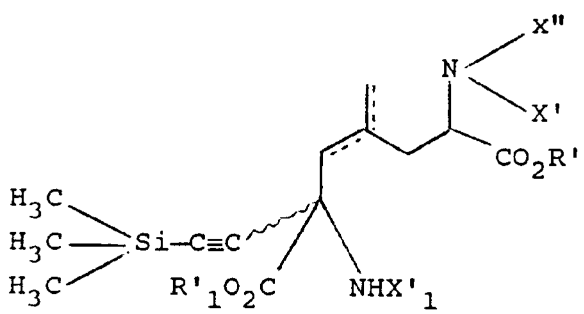

($IV_B$)

puis ou bien soumet le composé ($IV_A$) ou ($IV_B$) à l'action d'un agent de clivage sélectif des fonctions esters

et du radical triméthylsilyle pour obtenir le composé de formule ($I_A$) :

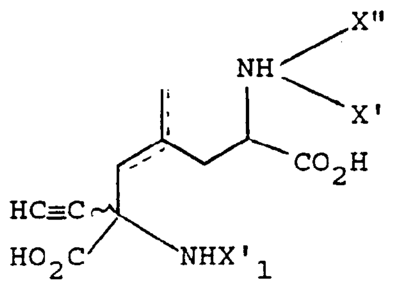

($I_A$)

que le cas échéant l'on soumet à l'action d'un agent de clivage du groupement protecteur d'amine pour obtenir le composé de formule ($I_B$) :

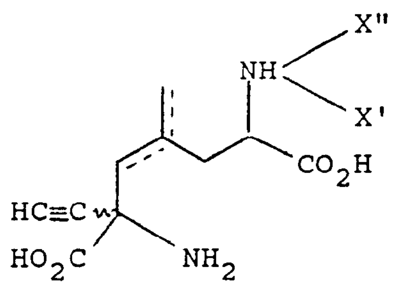

($I_B$)

ou bien soumet le composé de formule ($IV_A$) ou ($IV_B$) à l'action d'un agent de clivage du triméthylsilyle pour obtenir le composé de formule ($I_C$) :

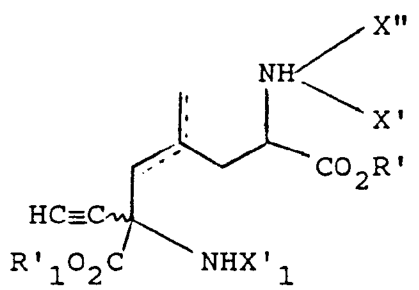

($I_C$)

puis l'on soumet si désiré, le composé de formule ($I_A$), ($I_B$) ou ($I_C$) à l'action d'un agent de réduction partielle pour obtenir le composé de formule ($I_D$) correspondant dans lequel Y représente un radical éthényle, ou à l'action d'agent de réduction totale de la triple liaison pour obtenir le composé de formule ($I_E$) correspondant dans lequel Y est un radical éthyle,
puis le cas échéant soumet le composé de formule (I) ainsi obtenu à la totalité ou à une partie seulement des opérations mentionnées à la fin de la revendication 1.

6. Procédé selon la revendication 5, caractérisé en ce que l'on soumet un composé de formule ($II_A$) telle que définie à la revendication 5, à l'action de l'azoture de sodium pour obtenir le composé de formule ($III_A$) telle que définie à la revendication 5, que l'on soumet à l'action d'un agent de réduction de l'azoture pour obtenir le composé de formule ($IV_A$) telle que définie à la revendication 5, puis ou bien soumet ce composé ($IV_A$) à l'action d'un agent de clivage sélectif des fonctions esters et du radical triméthylsilyle pour obtenir le composé de formule ($I'_A$) :

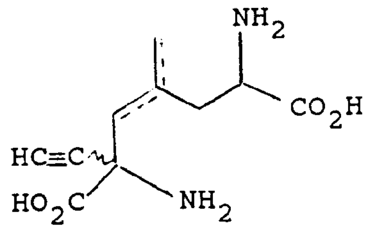

(I'$_A$)

que le cas échéant l'on soumet à l'action d'un agent de clivage du groupement protecteur d'amine pour obtenir le composé de formule (I'$_B$) :

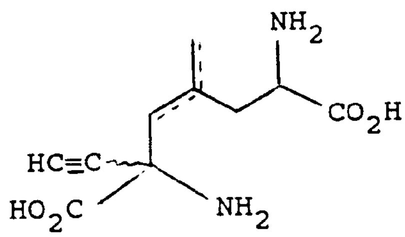

(I'$_B$)

ou bien soumet ce composé de formule (IV$_A$) à l'action d'un agent de clivage du triméthylsilyle pour obtenir le composé de formule (I'$_C$) :

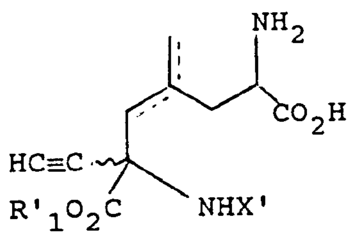

(I'$_C$)

puis soumet si désiré, le composé de formule (I'$_A$), (I'$_B$) ou (I'$_C$) à l'action d'un agent de réduction partielle pour obtenir le composé de formule (I'$_D$) correspondant dans lequel Y représente un radical éthényle, ou à l'action d'agent de réduction totale de la triple liaison pour obtenir le composé de formule (I'$_E$) correspondant dans lequel Y est un radical éthyle,
puis si désiré soumet chacun des composés de formule (I) ainsi obtenus à la totalité ou à une partie seulement des opérations mentionnées à la fin de la revendication 1.

**7.** Procédé selon la revendication 1, caractérisé en ce que l'on utilise au départ un composé de formule (II) dans laquelle les traits pointillés représentent une double liaison exo.

**8.** Procédé selon la revendication 1, caractérisé en ce que l'on utilise au départ une amine de formule

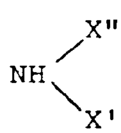

dans laquelle X' représente un atome d'hydrogène ou un radical méthyle et X" représente un radical méthyle ou benzyle.

**9.** Procédé selon la revendication 1, caractérisé en ce que l'on utilise au départ un composé de formule (II) dans laquelle Y' représente un radical acétylénique ou un précurseur d'un radical acétylénique.

**10.** Procédé selon la revendication 2, caractérisé en ce que l'on prépare l'un quelconque des produits de formule (I), dont les noms suivent :

- l'acide 2,6-diamino 2-éthényl 4-méthylène heptanedioïque,
- l'acide 2,6-diamino 2-éthyl 4-méthylène heptanedioïque,
- l'acide 2-amino 2-éthynyl 4-méthylène 6-(phénylméthyl) amino heptanedioïque.

**11.** Procédé de préparation de compositions pharmaceutiques, caractérisé en ce que l'on met, à titre de principe actif, l'un au moins des produits de formule (I) telle que définie à la revendication 1 ou l'un au moins de leurs sels avec les acides organiques ou minéraux ou avec les bases pharmaceutiquement acceptables, sos une forme destinée à cet usage.

**12.** Procédé de préparation de compositions pharmaceutiques, caractérisé en ce que l'on met, à titre de principe actif, l'un au moins des produits de formule (I') telle que définie à la revendication 2 ou l'un au moins de leurs sels avec les acides organiques ou minéraux ou avec les bases pharmaceutiquement acceptables, sous une forme destinée à cet usage.

**13.** Procédé de préparation de compositions pharmaceutiques, caractérisé en ce que l'on met, à titre de principe actif, l'un au moins des produits de formule (I) telle que définie à la revendication 10 ou l'un au moins de leurs sels avec les acides organiques ou minéraux ou avec les bases pharmaceutiquement acceptables, sous une forme destinée à cet usage.

**14.** A titre de produits chimiques nouveaux, les composés de formule (II) tels que définis à la revendication 1, dans lesquels Y' est un radical triméthylsilyle alkynyle renfermant jusqu'à 8 atomes de carbone.

**15.** A titre de produits chimiques nouveaux, les composés de formule (III) et ($III_A$) tels que définis aux revendications 4 et 5.

**Patentansprüche**

**Patentansprüche für folgende Vertragsstaaten : AT, BE, CH, DE, DK, FR, GB, IT, LI, LU, NL, SE**

**1.** Verbindungen der allgemeinen Formel (I)

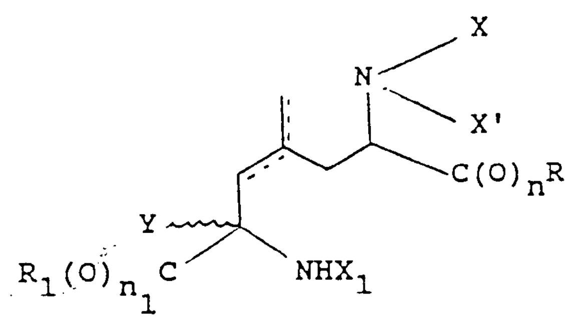

(I)

worin

die gestrichelten Linien eine etwaige endo- oder exo-Doppelbindung bedeuten,

Y für einen Alkyl-, Alkenyl- oder Alkinylrest mit 2 bis 18 Kohlenstoffatomen oder einen Alkylrest mit 1 bis 18 Kohlenstoffatomen, der durch ein oder mehrere Halogenatome substituiert ist, steht,

X, X' und $X_1$, identisch oder voneinander verschieden, ein Wasserstoffatom, einen Alkyl-, Alkenyl- oder Alkinylrest mit bis zu 8 Kohlenstoffatomen, einen Arylrest mit bis zu 14 Kohlenstoffatomen, einen Aralkylrest mit bis zu 18 Kohlenstoffatomen oder einen Acylrest, der sich von einer Fettsäure und/oder einer alpha- und/oder omega-Aminosäure ableitet, bedeuten, und

<u>entweder</u> n und/oder $n_1$ die Zahl 1 darstellen und R und/oder $R_1$ für den Rest eines Amins oder einer alpha- oder omega-Aminosäure stehen

<u>oder</u> n und/oder $n_1$ für die Zahl 2 stehen und R und $R_1$ ein Wasserstoffatom, einen Alkylrest mit bis zu 8 Kohlenstoffatomen oder einen Arylrest mit bis zu 14 Kohlenstoffatomen oder einen Aralkylrest mit bis zu 18 Kohlenstoffatomen oder einen Rest

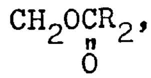

worin $R_2$ für einen Alkylrest mit bis zu 8 Kohlenstoffatomen oder einen Arylrest mit bis zu 14 Kohlenstoffatomen steht, bedeuten,
sowie deren Salze mit organischen oder Mineralsäuren oder mit Basen.

2. Verbindungen der Formel (I), wie in Anspruch 1 definiert, worin die gestrichelten Linien eine exo-Doppelbindung darstellen, sowie deren Salze mit organischen oder Mineralsäuren oder mit Basen.

3. Verbindungen der Formel (I), wie in Anspruch 1 oder 2 definiert, worin X' für ein Wasserstoffatom steht, sowie deren Salze mit organischen oder Mineralsäuren oder mit Basen.

4. Verbindungen der Formel (I), wie in einem der Ansprüche 1 bis 3 definiert, worin X, X' und $X_1$ jeweils ein Wasserstoffatom bedeuten, sowie deren Salze mit organischen oder Mineralsäuren oder mit Basen.

5. Verbindungen der Formel (I), wie in Anspruch 1 oder 2 definiert, worin X' für ein Wasserstoffatom oder einen Methylrest steht, X einen Methyl- oder Benzylrest bedeutet und $X_1$ ein Wasserstoffatom wiedergibt, sowie deren Salze mit organischen oder Mineralsäuren oder mit Basen.

6. Verbindungen der Formel (I), wie in einem der Ansprüche 1 bis 5 definiert, worin R und $R_1$ jeweils ein Wasserstoffatom bedeuten, sowie deren Salze mit organischen oder Mineralsäuren oder mit Basen.

7. Verbindungen der Formel (I), wie in einem der Ansprüche 1 bis 6 definiert, worin Y für einen acetylenischen Rest steht, sowie deren Salze mit organischen oder Mineralsäuren oder mit Basen.

8. Verbindungen der Formel (I), wie in einem der Ansprüche 1 bis 6 definiert, worin Y für einen ethylenischen Rest steht, sowie deren Additions-salze mit organischen oder Mineralsäuren oder mit Basen.

9. Verbindungen der Formel (I), wie in einem der Ansprüche 1 bis 6 definiert, worin Y für einen Ethylrest steht, sowie deren Salze mit organischen oder Mineralsäuren oder mit Basen.

10. Verbindungen der Formel (I), wie in Anspruch 1 definiert, mit den folgenden Bezeichnungen:
2,6-Diamino-2-ethenyl-4-methylen-heptandisäure,
2,6-Diamino-2-ethyl-4-methylen-heptandisäure,
2-Amino-2-ethinyl-4-methylen-6-(phenylmethyl)-aminoheptandisäure.

11. Als Arzneimittel die Verbindungen der Formel (I), wie in einem der Ansprüche 1 bis 9 definiert, sowie deren Salze mit pharmazeutisch verträglichen, organischen oder Mineralsäuren oder Basen.

12. Als Arzneimittel die Verbindungen der Formel (I), wie in Anspruch 10 definiert, sowie deren Salze mit pharmazeutisch verträglichen, organischen oder Mineralsäuren oder Basen.

13. Verfahren zur Herstellung der Verbindungen der Formel (I), wie in einem der Ansprüche 1 bis 10 definiert, dadurch gekennzeichnet, daß man eine Verbindung der allgemeinen Formel (II)

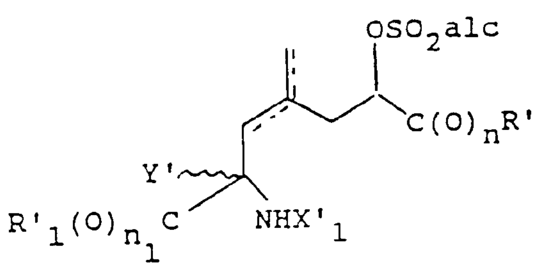

(II)

worin alc für einen Alkylrest mit 1 bis 8 Kohlenstoffatomen steht, der gegebenenfalls durch ein oder mehrere Halogenatome substituiert ist, n, $n_1$ und die gestrichelten Linien die vorstehend angegebene Bedeutung besitzen, Y' entweder Y oder einen Vorläufer von Y bedeutet, $X'_1$, R' und $R'_1$ die gleiche Bedeutung

wie $X_1$, R und $R_1$ mit Ausnahme von Wasserstoff haben, der Einwirkung eines Reagens, das befähigt ist, einen Vorläufer für die Aminofunktion zu liefern, oder eines Amins der Formel

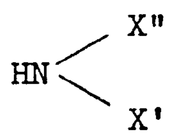

unterzieht, worin X′ die in Anspruch 1 angegebene Bedeutung besitzt und X″ die für X angegebene Bedeutung mit Ausnahme von Wasserstoff besitzt, um gegebenenfalls nach geeigneter Behandlung die Verbindung der Formel (I) zu erhalten, die man gewünschtenfalls der Gesamtheit oder lediglich eines Teils der folgenden Maßnahmen in beliebiger Reihenfolge unterzieht:

Entfernung der Schutzgruppen der Aminofunktionen,
Funktionalisierung der Aminofunktionen,
Hydrolyse der Esterfunktionen,
Behandlung von Y′, um Y zu erhalten,
vollständige oder selektive Reduktion des Substituenten Y, wenn dieser ungesättigt ist,
Salzbildung.

**14.** Verfahren gemäß Anspruch 13,dadurch gekennzeichnet, daß das Reagens, welches in der Lage ist, einen Vorläufer für die Aminofunktion zu liefern, Natriumazid, Kaliumphthalimid oder Hydroxylamin ist.

**15.** Verfahren gemäß Anspruch 13, dadurch gekennzeichnet, daß man eine Verbindung der Formel (II), wie in Anspruch 1 3 definiert, der Einwirkung von Natriumazid unterzieht, um zu der Verbindung der Formel (III)

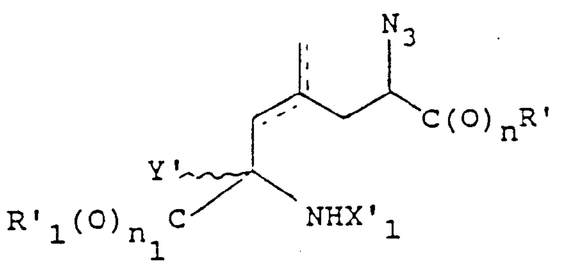

(III)

zu gelangen, welche man der Einwirkung eines Reduktionsmittels für die Gruppe $N_3$ unterzieht, um zu der Verbindung der Formel (IV)

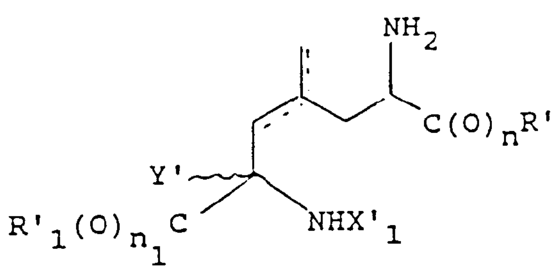

(IV)

zu gelangen, die man der Gesamtheit oder lediglich eines Teils der am Ende von Anspruch 13 erwähnten Maßnahmen unterzieht.

**16.** Verfahren gemäß Anspruch 13, dadurch gekennzeichnet, daß man eine Verbindung der Formel ($II_A$)

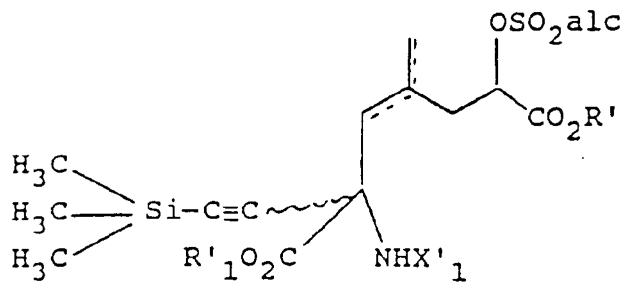

(II$_A$)

entweder der Einwirkung von Natriumazid unterzieht, um zu der Verbindung der Formel (III$_A$)

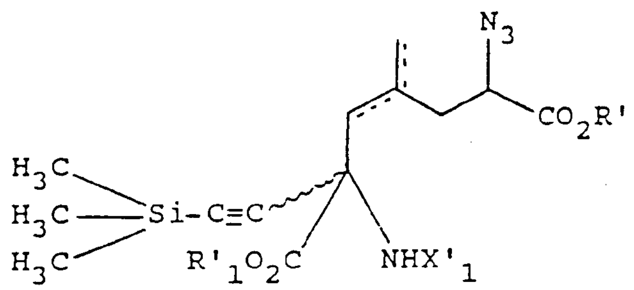

(III$_A$)

zu gelangen, welche man der Einwirkung eines Reduktionsmittels für das Azid unterzieht, um zu der Verbindung der Formel (IV$_A$)

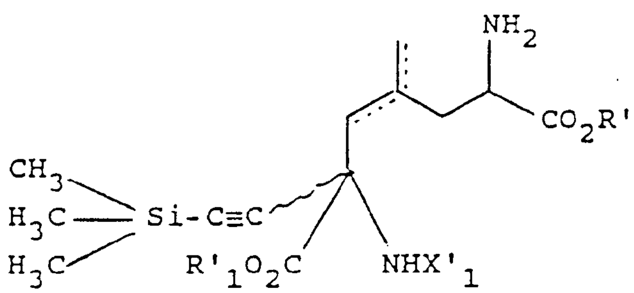

(IV$_A$)

zu gelangen,
oder der Einwirkung eines Amins der Formel

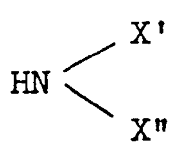

unterzieht, worin X′ und X″ die in Anspruch 13 angegebene Bedeutung besitzen, um zu der Verbindung der Formel (IV$_B$)

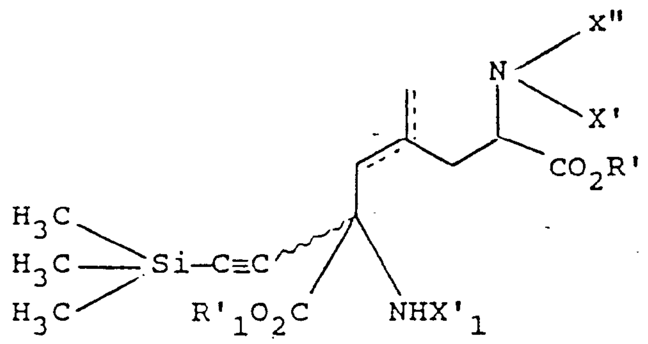

(IV$_B$)

zu gelangen, wonach man entweder die Verbindung der Formel (IV$_A$) oder (IV$_B$) der Einwirkung eines Mittels für die selektive Spaltung der Esterfunktionen und des Trimethylsilylrestes unterzieht, um zu der Verbindung der Formel (I$_A$)

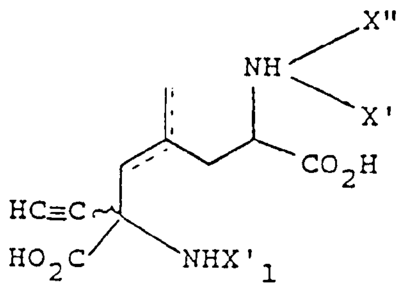

(I$_A$)

zu gelangen, die man gegebenenfalls der Einwirkung eines Mittels für die Abspaltung der Schutzgruppe des Amins unterzieht, um zu der Verbindung der Formel (I$_B$)

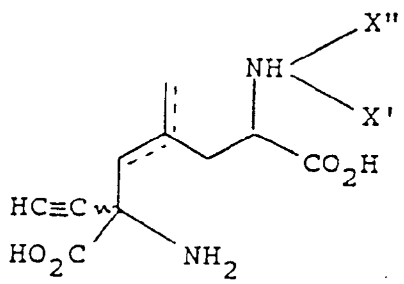

(I$_B$)

zu gelangen, oder die Verbindung der Formel (IV$_A$) oder (IV$_B$) der Einwirkung eines Mittels für die Abspaltung des Trimethylsilyls unterzieht, um zu der Verbindung der Formel (I$_C$)

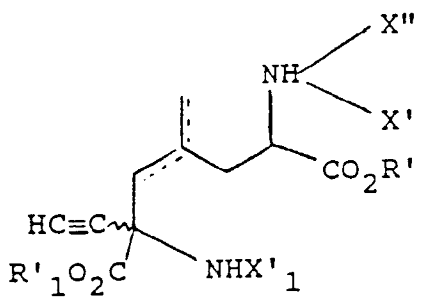

(I$_C$)

zu gelangen, wonach man gewünschtenfalls die Verbindung der Formel (I$_A$), (I$_B$) oder (I$_C$) der Einwirkung eines Mittels für die partielle Reduktion unterzieht, um die entsprechende Verbindung der Formel (I$_D$) zu erhalten, worin Y einen Ethenylrest bedeutet, oder der Einwirkung eines Mittels für die vollständige Reduktion der Dreifachbindung unterzieht, um zu der entsprechenden Verbindung der Formel ($I_E$) zu gelangen, worin Y einen Ethylrest bedeutet,
wonach man gegebenenfalls die so erhaltene Verbindung der Formel (I) der Gesamtheit oder lediglich eines Teils der am Ende von Anspruch 13 genannten Maßnahmen unterzieht.

**17.** Als neue chemische Produkte die Verbindungen der Formel (II), wie in Anspruch 13 definiert, worin Y′ für einen Trimethylsilylalkinylrest mit bis zu 8 Kohlenstoffatomen steht.

**18.** Als neue chemische Produkte die Verbindungen der Formel (III) und ($III_A$), wie in Anspruch 15 und 16 definiert.

**Patentansprüche für folgenden Vertragsstaat : ES**

**1.** Verfahren zur Herstellung von Verbindungen der allgemeinen Formel (I)

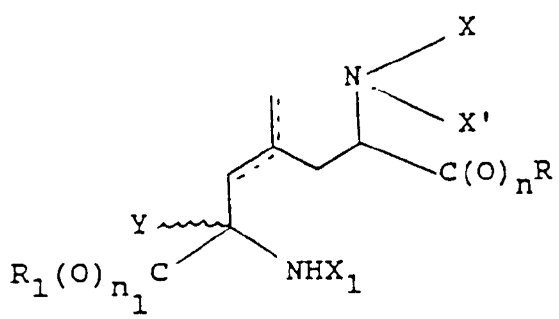

(I)

worin

die gestrichelten Linien eine etwaige endo- oder exoDoppelbindung bedeuten,

Y für einen Alkyl-, Alkenyl- oder Alkinylrest mit 2 bis 18 Kohlenstoffatomen oder einen Alkylrest mit 1 bis 18 Kohlenstoffatomen, der durch ein oder mehrere Halogenatome substituiert ist, steht,

X, X′ und $X_1$, identisch oder voneinander verschieden, ein Wasserstoffatom, einen Alkyl-, Alkenyl- oder Alkinylrest mit bis zu 8 Kohlenstoffatomen, einen Arylrest mit bis zu 14 Kohlenstoffatomen, einen Aralkylrest mit bis zu 18 Kohlenstoffatomen oder einen Acylrest, der sich von einer Fettsäure und/oder einer alpha- und/oder omega-Aminosäure ableitet, bedeuten, und

entweder n und/oder $n_1$ die Zahl 1 darstellen und R und/oder R1 für den Rest eines Amins oder einer alpha- oder omega-Aminosäure stehen

oder n und/oder $n_1$ für die Zahl 2 stehen und R und $R_1$ ein Wasserstoffatom, einen Alkylrest mit bis zu 8 Kohlenstoffatomen oder einen Arylrest mit bis zu 14 Kohlenstoffatomen oder einen Aralkylrest mit bis zu 18 Kohlenstoffatomen oder einen Rest

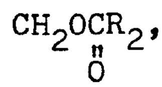

worin $R_2$ für einen Alkylrest mit bis zu 8 Kohlenstoffatomen oder einen Arylrest mit bis zu 14 Kohlenstoffatomen steht, bedeuten, sowie von deren Salzen mit organischen oder Mineralsäuren oder mit Basen, dadurch gekennzeichnet, daß man eine Verbindung der allgemeinen Formel (II)

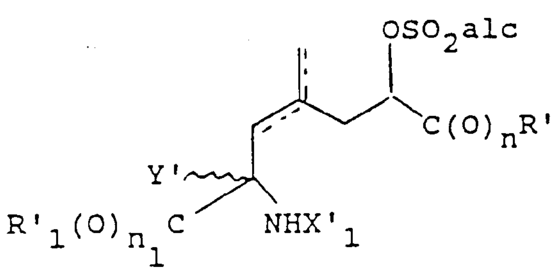

(II)

worin alc für einen Alkylrest mit 1 bis 8 Kohlenstoffatomen steht, der gegebenenfalls durch ein oder mehrere Halogenatome substituiert ist, n, $n_1$ und die gestrichelten Linien die vorstehend angegebene Bedeutung besitzen, Y′ entweder Y oder einen Vorläufer von Y bedeutet, $X'_1$, R′ und $R'_1$ die gleiche Bedeutung wie $X_1$, R und $R_1$ mit Ausnahme von Wasserstoff haben, der Einwirkung eines Reagens, das befähigt ist, einen Vorläufer für die Aminofunktion zu liefern, oder eines Amins der Formel

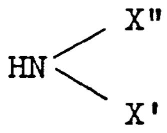

unterzieht, worin X′ die vorstehend angegebene Bedeutung besitzt und X″ die für X angegebene Bedeutung mit Ausnahme von Wasserstoff besitzt, um gegebenenfalls nach geeigneter Behandlung die Verbindung der Formel (I) zu erhalten, die man gewünschtenfalls der Gesamtheit oder lediglich eines Teils der folgenden Maßnahmen in beliebiger Reihenfolge unterzieht:

Entfernung der Schutzgruppen der Aminofunktionen,
Funktionalisierung der Aminofunktionen,
Hydrolyse der Esterfunktionen,
Behandlung von Y′, um Y zu erhalten,
vollständige oder selektive Reduktion des Substituenten Y, wenn dieser ungesättigt ist,
Salzbildung.

2. Verfahren gemäß Anspruch 1 zur Herstellung der Produkte der Formel (I), wie in Anspruch 1 definiert, mit der Formel (I′)

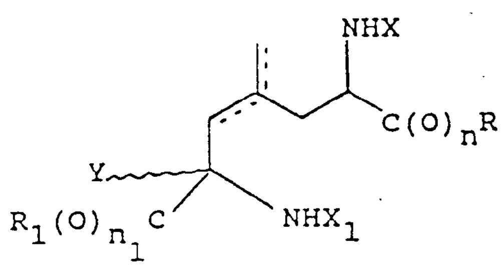

(I')

worin X, $X_1$, R, $R_1$, n, $n_1$ und Y die vorstehend angegebene Bedeutung besitzen, dadurch gekennzeichnet, daß man mit der Verbindung der Formel (II) ein Reagens, das in der Lage ist, einen Vorläufer für die Aminofunktion zu liefern, oder ein Amin der Formel

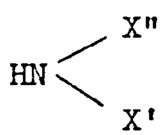

worin X″ die vorstehend angegebene Bedeutung hat und X′ für ein Wasserstoffatom steht, umsetzt.

3. Verfahren gemäß Anspruch 2, dadurch gekennzeichnet, daß das Reagens, welches in der Lage ist, einen Vorläufer für die Aminofunktion zu liefern, Natriumazid, Kaliumphthalimid oder Hydroxylamin ist.

4. Verfahren gemäß Anspruch 2, dadurch gekennzeichnet, daß man eine Verbindung der Formel (II), wie in Anspruch 1 definiert, der Einwirkung von Natriumazid unterzieht, um zu der Verbindung der Formel (III)

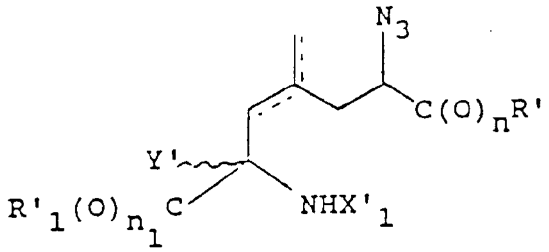

(III)

zu gelangen, welche man der Einwirkung eines Reduktionsmittels für die Gruppe $N_3$ unterzieht, um zu der Verbindung der Formel (IV)

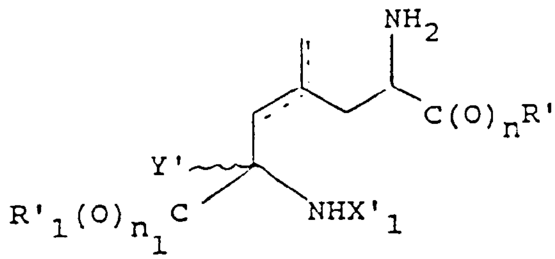

(IV)

zu gelangen, die man der Gesamtheit oder lediglich eines Teils der am Ende von Anspruch 1 erwähnten Maßnahmen unterzieht.

**5.** Verfahren gemäß Anspruch 1, dadurch gekennzeichnet, daß man eine Verbindung der Formel ($II_A$)

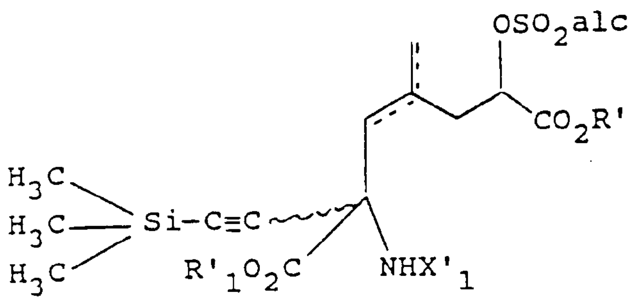

($II_A$)

entweder der Einwirkung von Natriumazid unterzieht, um zu der Verbindung der Formel ($III_A$)

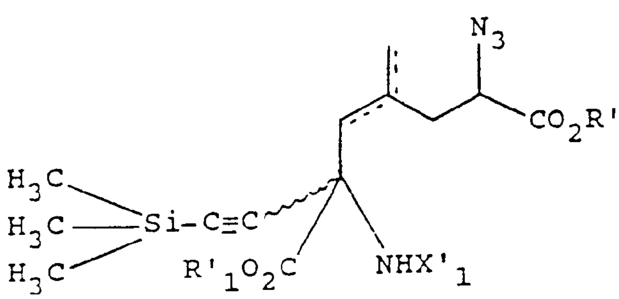

($III_A$)

zu gelangen, welche man der Einwirkung eines Reduktionsmittels für das Azid unterzieht, um zu der Verbindung der Formel ($IV_A$)

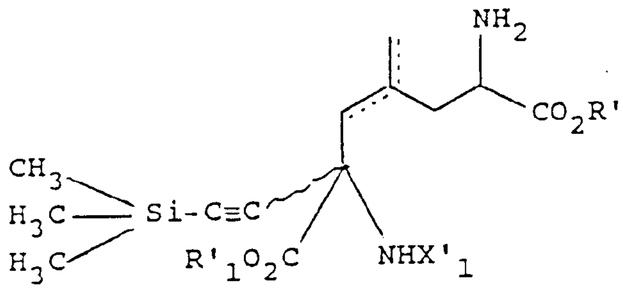

($IV_A$)

zu gelangen,
oder der Einwirkung eines Amins der Formel

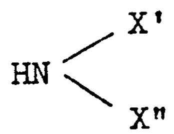

unterzieht, worin X′ und X″ die in Anspruch 1 angegebene Bedeutung besitzen, um zu der Verbindung der Formel ($IV_B$)

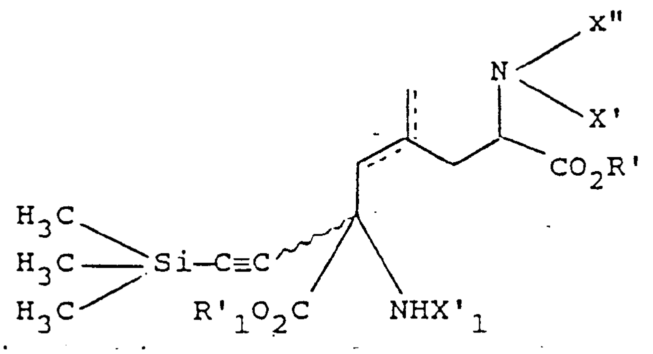

($IV_B$)

zu gelangen, wonach man entweder die Verbindung der Formel ($IV_A$) oder ($IV_B$) der Einwirkung eines Mittels für die selektive Spaltung der Esterfunktionen und des Trimethylsilylrestes unterzieht, um zu der Verbindung der Formel ($I_A$)

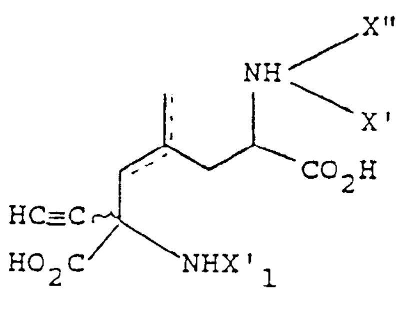

($I_A$)

zu gelangen, die man gegebenenfalls der Einwirkung eines Mittels für die Abspaltung der Schutzgruppe des Amins unterzieht, um zu der Verbindung der Formel ($I_B$)

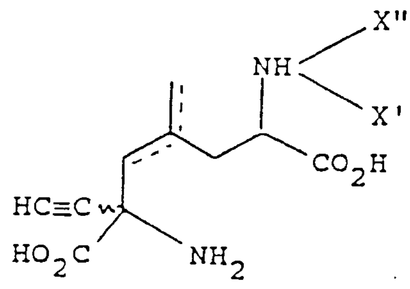

($I_B$)

zu gelangen, <u>oder</u> die Verbindung der Formel ($IV_A$) oder ($IV_B$) der Einwirkung eines Mittels für die Abspaltung des Trimethylsilyls unterzieht, um zu der Verbindung der Formel ($I_C$)

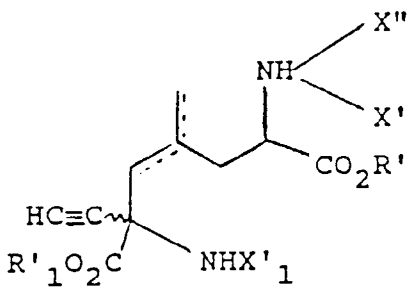

($I_C$)

zu gelangen, wonach man gewünschtenfalls die Verbindung der Formel ($I_A$), ($I_B$) oder ($I_C$) der Einwirkung eines Mittels für die partielle Reduktion unterzieht, um die entsprechende Verbindung der Formel ($I_D$) zu erhalten, worin Y einen Ethenylrest bedeutet, oder der Einwirkung eines Mittels für die vollständige Reduktion der Dreifachbindung unterzieht, um zu der entsprechenden Verbindung der Formel ($I_E$) zu gelangen, worin Y einen Ethylrest bedeutet,
wonach man gegebenenfalls die so erhaltene Verbindung der Formel (I) der Gesamtheit oder lediglich eines Teils der am Ende von Anspruch 1 genannten Maßnahmen unterzieht.

6. Verfahren gemäß Anspruch 5 , dadurch gekennzeichnet, daß man eine Verbindung der Formel ($II_A$), wie in Anspruch 5 definiert, der Einwirkung von Natriumazid unterzieht, um zu der Verbindung der Formel ($III_A$), wie in Anspruch 5 definiert, zu gelangen, welche man der Einwirkung eines Mittels für die Reduktion des Azids unterzieht, um zu der Verbindung der Formel ($IV_A$), wie in Anspruch 5 definiert, zu gelangen, wonach man <u>entweder</u> die Verbindung der Formel ($IV_A$) der Einwirkung eines Mittels für die selektive Spaltung der Esterfunktionen und des Trimethylsilylrestes unterzieht, um zu der Verbindung der Formel ($I'_A$)

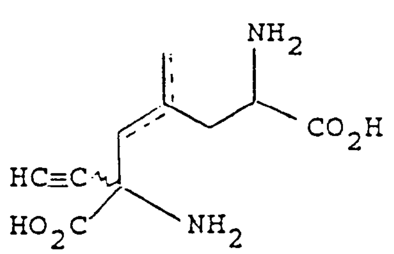

($I'_A$)

zu gelangen, die man gegebenenfalls der Einwirkung eines Mittels für die Abspaltung der Schutzgruppe des Amins unterzieht, um zu der Verbindung der Formel ($I'_B$)

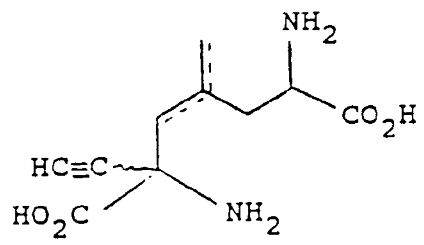

$(I'_B)$

zu gelangen, oder diese Verbindung der Formel ($IV_A$) der Einwirkung eines Mittels für die Abspaltung des Trimethylsilyls unterzieht, um zu der Verbindung der Formel ($I'_C$)

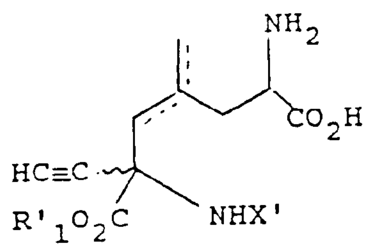

$(I'_C)$

zu gelangen, wonach man gewünschtenfalls die Verbindung der Formel ($I'_A$), ($I'_B$) oder ($I'_C$) der Einwirkung eines Mittels für die partielle Reduktion unterzieht, um die entsprechende Verbindung der Formel ($I'_D$) zu erhalten, worin Y für einen Ethenylrest steht, oder der Einwirkung eines Mittels für die vollständige Reduktion der Dreifachbindung unterzieht, um zu der entsprechenden Verbindung der Formel ($I'_E$) zu gelangen, worin Y für einen Ethylrest steht,
wonach man gewünschtenfalls eine jede der so erhaltenen Verbindungen der Formel (I) der Gesamtheit oder lediglich eines Teils der am Ende von Anspruch 1 genannten Maßnahmen unterzieht.

7. Verfahren gemäß Anspruch 1, dadurch gekennzeichnet, daß man von einer Verbindung der Formel (II) ausgeht, worin die gestrichelten Linien eine exo-Doppelbindung bedeuten.

8. Verfahren gemäß Anspruch 1, dadurch gekennzeichnet, daß man von einem Amin der Formel

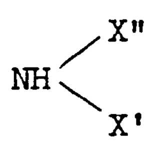

ausgeht, worin X′ ein Wasserstoffatom oder einen Methylrest bedeutet und X″ einen Methyl- oder Benzylrest darstellt.

9. Verfahren gemäß Anspruch 1, dadurch gekennzeichnet, daß man von einer Verbindung der Formel (II) ausgeht, worin Y′ einen acetylenischen Rest oder einen Vorläufer eines acetylenischen Restes bedeutet.

**Patentansprüche für folgenden Vertragsstaat : GR**

1. Verfahren zur Herstellung von Verbindungen der allgemeinen Formel (I)

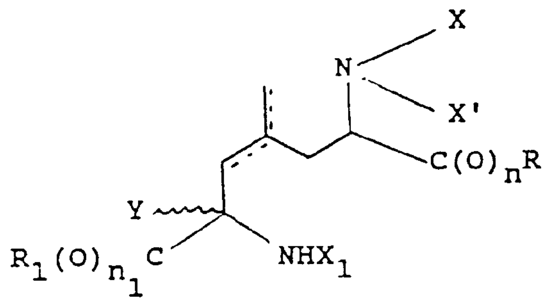

(I)

worin

die gestrichelten Linien eine etwaige endo- oder exo-Doppelbindung bedeuten,

Y für einen Alkyl-, Alkenyl- oder Alkinylrest mit 2 bis 18 Kohlenstoffatomen oder einen Alkylrest mit 1 bis 18 Kohlenstoffatomen, der durch ein oder mehrere Halogenatome substituiert ist, steht,

X, X′ und $X_1$, identisch oder voneinander verschieden, ein Wasserstoffatom, einen Alkyl-, Alkenyl- oder Alkinylrest mit bis zu 8 Kohlenstoffatomen, einen Arylrest mit bis zu 14 Kohlenstoffatomen, einen Aralkylrest mit bis zu 18 Kohlenstoffatomen oder einen Acylrest, der sich von einer Fettsäure und/oder einer alpha- und/oder omega-Aminosäure ableitet, bedeuten, und

entweder n und/oder $n_1$ die Zahl 1 darstellen und R und/oder $R_1$ für den Rest eines Amins oder einer alpha- oder omega-Aminosäure stehen

oder n und/oder $n_1$ für die Zahl 2 stehen und R und $R_1$ ein Wasserstoffatom, einen Alkylrest mit bis zu 8 Kohlenstoffatomen oder einen Arylrest mit bis zu 14 Kohlenstoffatomen oder einen Aralkylrest mit bis zu 18 Kohlenstoffatomen oder einen Rest

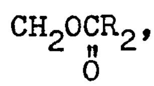

worin $R_2$ für einen Alkylrest mit bis zu 8 Kohlenstoffatomen oder einen Arylrest mit bis zu 14 Kohlenstoffatomen steht, bedeuten, sowie von deren Salzen mit organischen oder Mineralsäuren oder mit Basen, dadurch gekennzeichnet, daß man eine Verbindung der allgemeinen Formel (II)

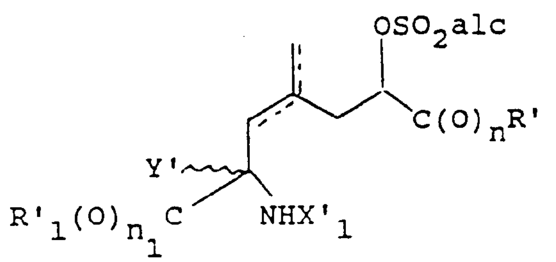

(II)

worin alc für einen Alkylrest mit 1 bis 8 Kohlenstoffatomen steht, der gegebenenfalls durch ein oder mehrere Halogenatome substituiert ist, n, $n_1$ und die gestrichelten Linien die vorstehend angegebene Bedeutung besitzen, Y′ entweder Y oder einen Vorlaufer von Y bedeutet, $X'_1$, R′ und $R'_1$ die gleiche Bedeutung wie $X_1$, R und $R_1$ mit Ausnahme von Wasserstoff haben, der Einwirkung eines Reagens, das befähigt ist, einen Vorläufer für die Aminofunktion zu liefern, oder eines Amins der Formel

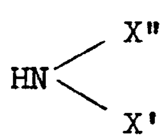

unterzieht, worin X′ die vorstehend angegebene Bedeutung besitzt und X″ die für X angegebene Bedeutung mit Ausnahme von Wasserstoff besitzt, um gegebenenfalls nach geeigneter Behandlung die Verbindung der Formel (I) zu erhalten, die man gewünschtenfalls der Gesamtheit oder lediglich eines Teils der

folgenden Maßnahmen in beliebiger Reihenfolge unterzieht:

Entfernung der Schutzgruppen der Aminofunktionen,
Funktionalisierung der Aminofunktionen,
Hydrolyse der Esterfunktionen,
Behandlung von Y', um Y zu erhalten,
vollständige oder selektive Reduktion des Substituenten Y, wenn dieser ungesättigt ist,
Salzbildung.

**2.** Verfahren gemäß Anspruch 1 zur Herstellung der Produkte der Formel (I), wie in Anspruch 1 definiert, mit der Formel (I')

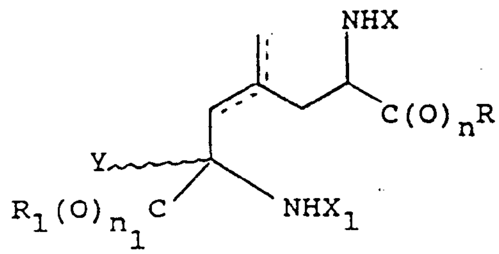

(I')

worin X, $X_1$, R, $R_1$, n, $n_1$ und Y die vorstehend angegebene Bedeutung besitzen, dadurch gekennzeichnet, daß man mit der Verbindung der Formel (II) ein Reagens, das in der Lage ist, einen Vorläufer für die Aminofunktion zu liefern, oder ein Amin der Formel

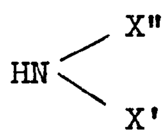

worin X" die vorstehend angegebene Bedeutung hat und X' für ein Wasserstoffatom steht, umsetzt.

**3.** Verfahren gemäß Anspruch 2, dadurch gekennzeichnet, daß das Reagens, welches in der Lage ist, einen Vorläufer für die Aminofunktion zu liefern, Natriumazid, Kaliumphthalimid oder Hydroxylamin ist.

**4.** Verfahren gemäß Anspruch 2, dadurch gekennzeichnet, daß man eine Verbindung der Formel (II), wie in Anspruch 1 definiert, der Einwirkung von Natriumazid unterzieht, um zu der Verbindung der Formel (III)

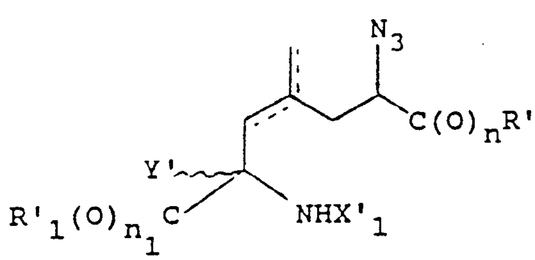

(III)

zu gelangen, welche man der Einwirkung eines Reduktionsmittels für die Gruppe $N_3$ unterzieht, um zu der Verbindung der Formel (IV)

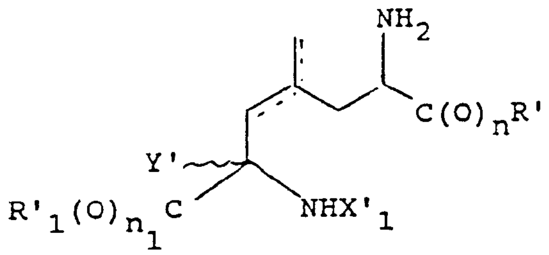

(IV)

zu gelangen, die man der Gesamtheit oder lediglich eines Teils der am Ende von Anspruch 1 erwähnten Maßnahmen unterzieht.

5. Verfahren gemäß Anspruch 1, dadurch gekennzeichnet, daß man eine Verbindung der Formel ($II_A$)

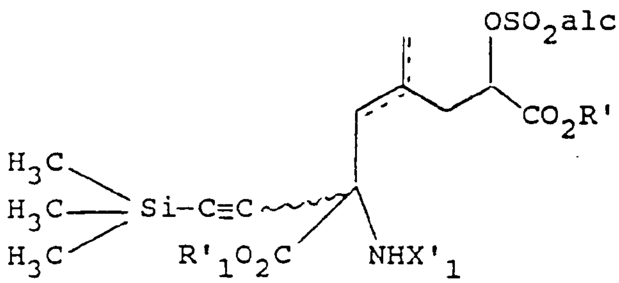

($II_A$)

entweder der Einwirkung von Natriumazid unterzieht, um zu der Verbindung der Formel ($III_A$)

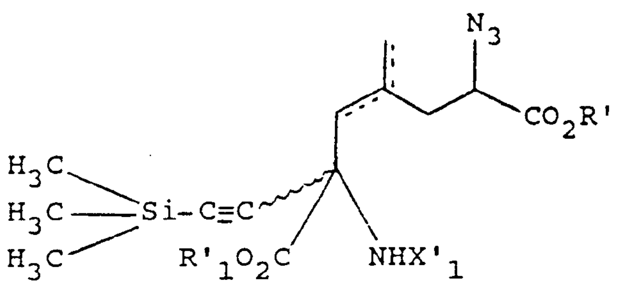

($III_A$)

zu gelangen, welche man der Einwirkung eines Reduktionsmittels für das Azid unterzieht, um zu der Verbindung der Formel ($IV_A$)

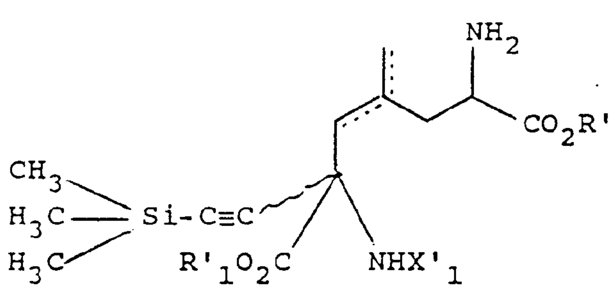

($IV_A$)

zu gelangen,
oder der Einwirkung eines Amins der Formel

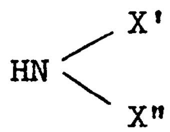

unterzieht, worin X′ und X″ die in Anspruch 1 angegebene Bedeutung besitzen, um zu der Verbindung der Formel ($IV_B$)

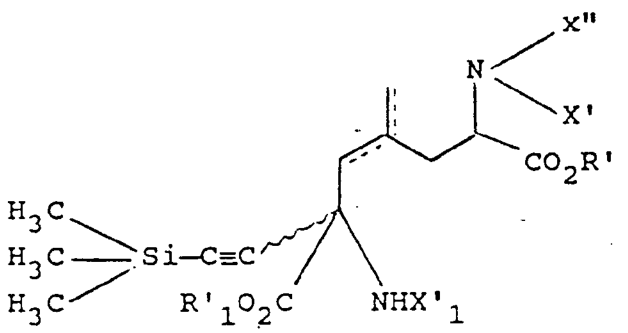

($IV_B$)

zu gelangen, wonach man entweder die Verbindung der Formel ($IV_A$) oder ($IV_B$) der Einwirkung eines Mittels für die selektive Spaltung der Esterfunktionen und des Trimethylsilylrestes unterzieht, um zu der Verbindung der Formel ($I_A$)

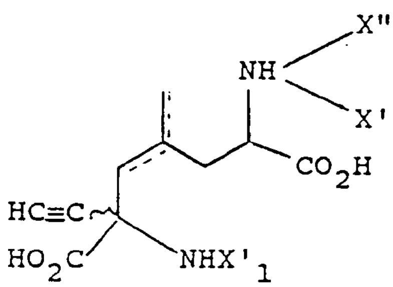

($I_A$)

zu gelangen, die man gegebenenfalls der Einwirkung eines Mittels für die Abspaltung der Schutzgruppe des Amins unterzieht, um zu der Verbindung der Formel ($I_B$)

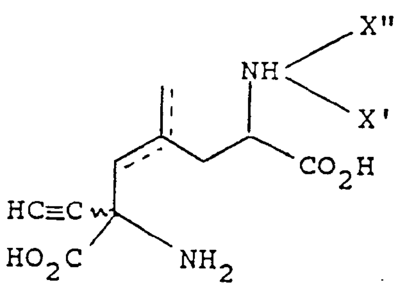

($I_B$)

zu gelangen, oder die Verbindung der Formel ($IV_A$) oder ($IV_B$) der Einwirkung eines Mittels für die Abspaltung des Trimethylsilyls unterzieht, um zu der Verbindung der Formel ($I_C$)

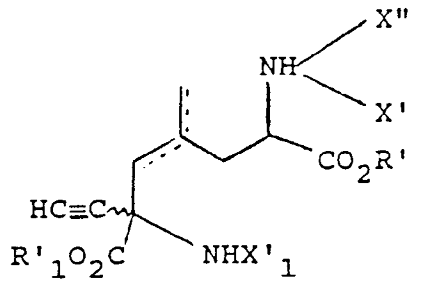

$(I_C)$

zu gelangen, wonach man gewünschtenfalls die Verbindung der Formel ($I_A$), ($I_B$) oder ($I_C$) der Einwirkung eines Mittels für die partielle Reduktion unterzieht, um die entsprechende Verbindung der Formel ($I_D$) zu erhalten, worin Y einen Ethenylrest bedeutet, oder der Einwirkung eines Mittels für die vollständige Reduktion der Dreifachbindung unterzieht, um zu der entsprechenden Verbindung der Formel ($I_E$) zu gelangen, worin Y einen Ethylrest bedeutet,
wonach man gegebenenfalls die so erhaltene Verbindung der Formel (I) der Gesamtheit oder lediglich eines Teils der am Ende von Anspruch 1 genannten Maßnahmen unterzieht.

**6.** Verfahren gemäß Anspruch 5 , dadurch gekennzeichnet, daß man eine Verbindung der Formel ($II_A$), wie in Anspruch 5 definiert, der Einwirkung von Natriumazid unterzieht, um zu der Verbindung der Formel ($III_A$), wie in Anspruch 5 definiert, zu gelangen, welche man der Einwirkung eines Mittels für die Reduktion des Azids unterzieht, um zu der Verbindung der Formel ($IV_A$), wie in Anspruch 5 definiert, zu gelangen, wonach man entweder die Verbindung der Formel ($IV_A$) der Einwirkung eines Mittels für die selektive Spaltung der Esterfunktionen und des Trimethylsilylrestes unterzieht, um zu der Verbindung der Formel ($I'_A$)

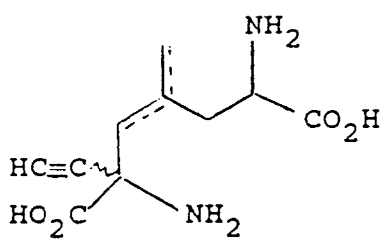

$(I'_A)$

zu gelangen, die man gegebenenfalls der Einwirkung eines Mittels für die Abspaltung der Schutzgruppe des Amins unterzieht, um zu der Verbindung der Formel ($I'_B$)

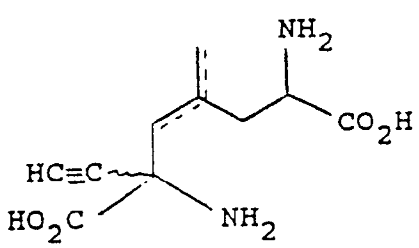

$(I'_B)$

zu gelangen, oder diese Verbindung der Formel ($IV_A$) der Einwirkung eines Mittels für die Abspaltung des Trimethylsilyls unterzieht, um zu der Verbindung der Formel ($I'_C$)

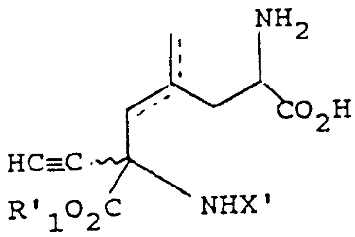

$(I'_C)$

zu gelangen, wonach man gewünschtenfalls die Verbindung der Formel $(I'_A)$, $(I'_B)$ oder $(I'_C)$ der Einwirkung eines Mittels für die partielle Reduktion unterzieht, um die entsprechende Verbindung der Formel $(I'_D)$ zu erhalten, worin Y für einen Ethenylrest steht, oder der Einwirkung eines Mittels für die vollständige Reduktion der Dreifachbindung unterzieht, um zu der entsprechenden Verbindung der Formel $(I'_E)$ zu gelangen, worin Y für einen Ethylrest steht,
wonach man gewünschtenfalls eine jede der so erhaltenen Verbindungen der Formel (I) der Gesamtheit oder lediglich eines Teils der am Ende von Anspruch 1 genannten Maßnahmen unterzieht.

**7.** Verfahren gemäß Anspruch 1, dadurch gekennzeichnet, daß man von einer Verbindung der Formel (II) ausgeht, worin die gestrichelten Linien eine exo-Doppelbindung bedeuten.

**8.** Verfahren gemäß Anspruch 1, dadurch gekennzeichnet, daß man von einem Amin der Formel

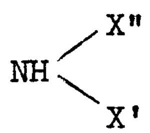

ausgeht, worin X′ ein Wasserstoffatom oder einen Methylrest bedeutet und X″ einen Methyl- oder Benzylrest darstellt.

**9.** Verfahren gemäß Anspruch 1, dadurch gekennzeichnet, daß man von einer Verbindung der Formel (II) ausgeht, worin Y′ einen acetylenischen Rest oder einen Vorläufer eines acetylenischen Restes bedeutet.

**10.** Verfahren gemäß Anspruch 2, dadurch gekennzeichnet, daß man eines der Produkte der Formel (I) mit den folgenden Bezeichnungen herstellt:
2,6-Diamino-2-ethenyl-4-methylen-heptandisäure,
2,6-Diamino-2-ethyl-4-methylen-heptandisäure,
2-Amino-2-ethinyl-4-methylen-6-(phenylmethyl)-aminoheptandisäure.

**11.** Verfahren zur Herstellung von pharmazeutischen Zusammensetzungen, dadurch gekennzeichnet, daß man als Wirkstoff zumindest eines der Produkte der Formel (I), wie in Anspruch 1 definiert, oder zumindest eines ihrer Salze mit pharmazeutisch verträglichen organischen oder Mineralsäuren oder Basen in eine für diese Verwendung bestimmte Form überführt.

**12.** Verfahren zur Herstellung von pharmazeutischen Zusammensetzungen, dadurch gekennzeichnet, daß man als Wirkstoff zumindest eines der Produkte der Formel (I′), wie in Anspruch 2 definiert, oder zumindest eines ihrer Salze mit pharmazeutisch verträglichen organischen oder Mineralsäuren oder Basen in eine für diese Verwendung bestimmte Form überführt.

**13.** Verfahren zur Herstellung von pharmazeutischen Zusammensetzungen, dadurch gekennzeichnet, daß man als Wirkstoff zumindest eines der Produkte der Formel (I), wie in Anspruch 10 definiert, oder zumindest eines ihrer Salze mit pharmazeutisch verträglichen organischen oder Mineralsäuren oder Basen in eine für diese Verwendung bestimmte Form überführt.

**14.** Als neue chemische Produkte die Verbindungen der Formel (II), wie in Anspruch 1 definiert, worin Y′ für einen Trimethylsilylalkinylrest mit bis zu 8 Kohlenstoffatomen steht.

**15.** Als neue chemische Produkte die Verbindungen der Formel (III) und ($III_A$), wie in Anspruch 4 und 5 definiert.

## Claims

### Claims for the following Contracting States : AT, BE, CH, DE, DK, FR, GB, IT, LI, LU, NL, SE

**1.** The compounds of general formula (I):

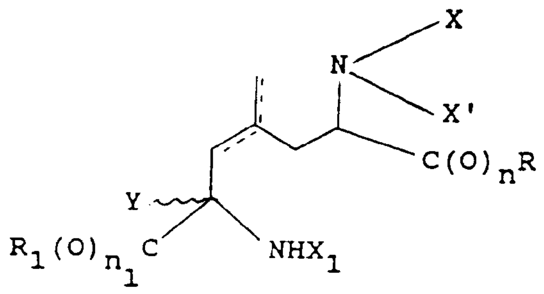

(I)

in which

- the dotted lines represent an optional endo or oxo double bond,
- Y represents an alkyl, alkenyl or alkynyl radical containing 2 to 18 carbon atoms, or an alkyl radical containing 1 to 18 carbon atoms substituted by one or more halogen atoms,
- X, X′ and $X_1$, identical or different, represent a hydrogen atom, an alkyl, alkenyl or alkynyl radical containing up to 8 carbon atoms, an aryl radical containing up to 14 carbon atoms, an aralkyl radical containing up to 18 carbon atoms, or an acyl radical derived from a fatty acid and/or from an alpha and/or omega amino acid,

and either n and/or $n_1$ represent the number 1 and R and/or $R_1$ represent the remainder of an amine or of an alpha or omega amino acid, or n and/or $n_1$ represent the number 2 and R and $R_1$ represent a hydrogen atom, an alkyl radical containing up to 8 carbon atoms or an aryl radical containing up to 14 carbon atoms, or an aralkyl radical containing up to 18 carbon atoms, or a

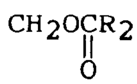

radical, in which $R_2$ represents an alkyl radical containing up to 8 carbon atoms, or an aryl radical containing up to 14 carbon atoms,as well as their salts with organic or mineral acids or with bases.

**2.** The compounds of formula (I) as defined in claim 1, in which the dotted lines represent an exo double bond, as well as their salts with organic or mineral acids or with bases.

**3.** The compounds of formula (I) as defined in claim 1 or 2, in which X′ represents a hydrogen atom, as well as their salts with organic or mineral acids or with bases.

**4.** The compounds of formula (I) as defined in any one of claims 1 to 3, in which X, X′ and $X_1$ each represent a hydrogen atom, as well as their salts with organic or mineral acids, or with bases.

**5.** The compounds of formula (I) as defined in claim 1 or 2, in which X′ represents a hydrogen atom or a methyl radical, X represents a methyl or benzyl radical and $X_1$ represents a hydrogen atom, as well as their salts with organic or mineral acids, or with bases.

**6.** The compounds of formula (I) as defined in any one of claims 1 to 5, in which R and $R_1$ each represent a hydrogen atom, as well as their salts with organic or mineral acids, or with bases.

**7.** The compounds of formula (I) as defined in any one of claims 1 to 6, in which Y represents an acetylenic radical as well as their salts with organic or mineral acids or with bases.

8. The compounds of formula (I) as defined in any one of claims 1 to 6, in which Y represents an ethylenic radical, as well as their addition salts with organic or mineral acids, or with bases.

9. The compounds of formula (I) as defined in any one of claims 1 to 6, in which Y represents an ethyl radical, as well as their salts with organic or mineral acids, or with bases.

10. The compounds of formula (I) as defined in claim 1, of which the names follow:
- 2,6-diamino 2-ethenyl 4-methylene heptanedioic acid,
- 2,6-diamino 2-ethyl 4-methylene heptanedioic acid,
- 2-amino 2-ethynyl 4-methylene 6-(phenylmethyl) amino heptanedioic acid.

11. As medicaments, the compounds of formula (I) as defined in any one of claims 1 to 9, as well as their salts with pharmaceutically acceptable organic or mineral acids or with pharmaceutically acceptable bases.

12. As medicaments, the compounds of formula (I) as defined in claim 10, as well as their salts with pharmaceutically acceptable organic or mineral acids or with pharmaceutically acceptable bases.

13. Preparation process for the compounds of formula (I) as defined in any one of claims 1 to 10, characterized in that a compound of general formula (II):

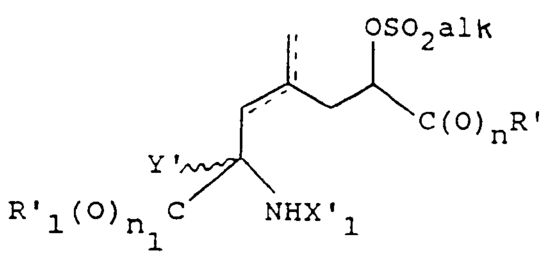

(II)

in which alk represents an alkyl radical containing 1 to 8 carbon atoms optionally substituted by one or more halogen atoms, n, $n_1$ and the dotted lines keep the same meaning as previously, Y′ represents either Y, or a precursor of Y, $X'_1$, R′ and $R'_1$ have the same meaning as $X_1$, R and $R_1$ with the exception of hydrogen, is subjected to the action of a reagent capable of providing a precursor of the amino function or of an amine of formula

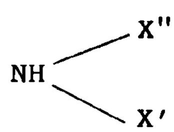

in which X′ has the meaning indicated in claim 1 and X″ has the meaning indicated for X with the exception of hydrogen, in order to obtain, if appropriate after suitable treatment, the compound of formula (I) which is subjected, if desired, to all or one part only of the following operations in any order:
- deprotection of the amine functions,
- functionalization of the amine functions,
- hydrolysis of the ester functions,
- treatment of Y′ in order to obtain Y,
- total or selective reduction of the Y substituent when this is unsaturated,
- salification.

14. Process according to claim 13, characterized in that the reagent capable of providing a precursor of the amino function is sodium nitride, potassium phthalimide or hydroxylamine.

15. Process according to claim 13, characterized in that a compound of formula (II) defined as in claim 13 is subjected to the action of sodium nitride in order to obtain the compound of formula (III):

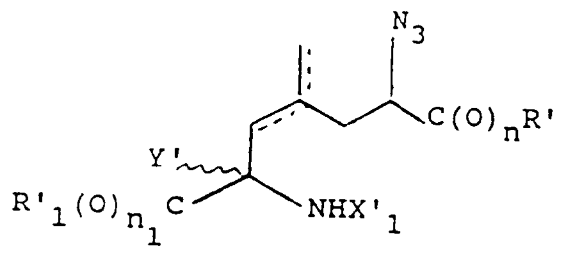

(III)

which is subjected to the action of an agent which reduces the $N_3$ group, in order to obtain the compound of formula (IV):

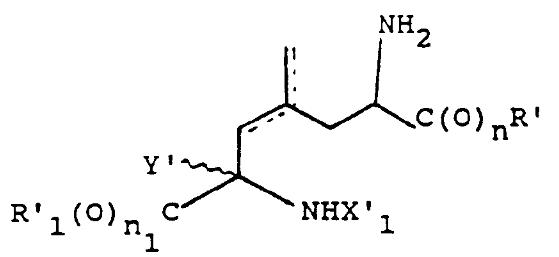

(IV)

which is subjected to all or one part only of the operations mentioned at the end of claim 13.

**16.** Process according to claim 13, characterized in that a compound of formula ($II_A$):

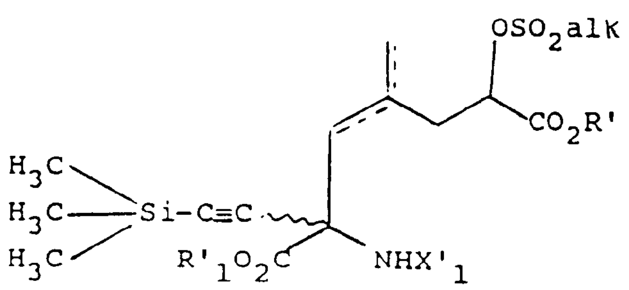

($II_A$)

is subjected

either to the action of sodium nitride in order to obtain the compound of formula ($III_A$):

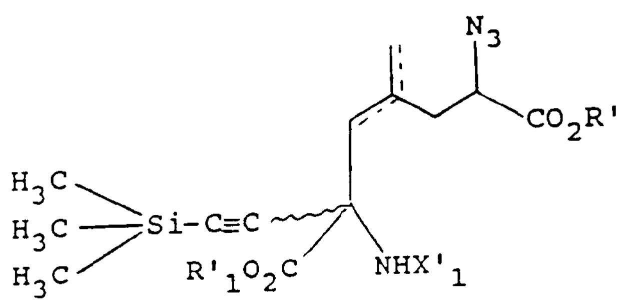

($III_A$)

which is subjected to the action of an agent which reduces the nitride, in order to obtain the compound of formula ($IV_A$):

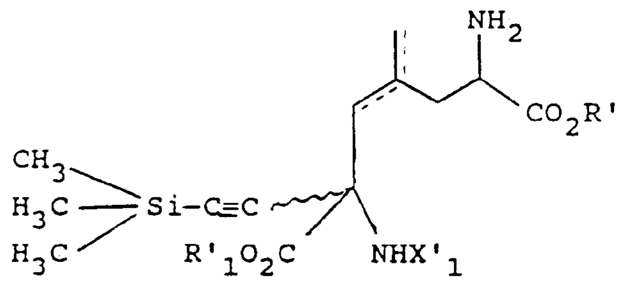

(IV$_A$)

or to the action of an amine of formula

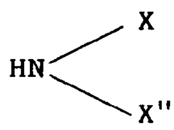

in which X′ and X″ have the meaning indicated in claim 13, in order to obtain the compound of formula (IV$_B$):

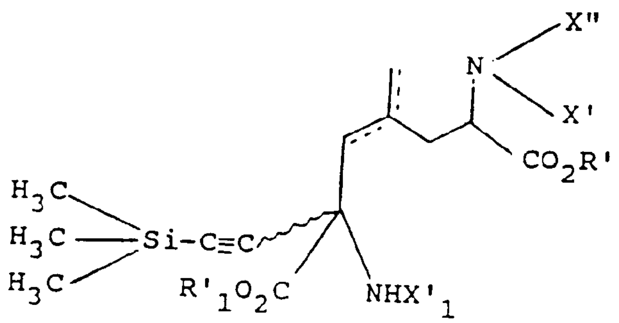

(IV$_B$)

then either compound (IV$_A$) or (IV$_B$) is subjected to the action of an agent which selectively cleaves the ester functions and the trimethylsilyl radical in order to obtain the compound of formula (I$_A$):

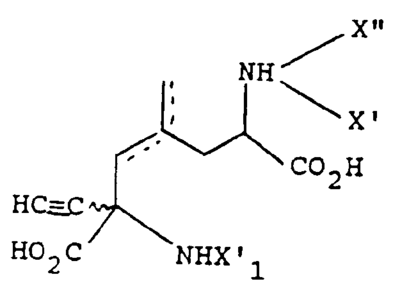

(I$_A$)

which if appropriate is subjected to the action of an agent which cleaves the amine protector group in order to obtain the compound of formula (I$_B$):

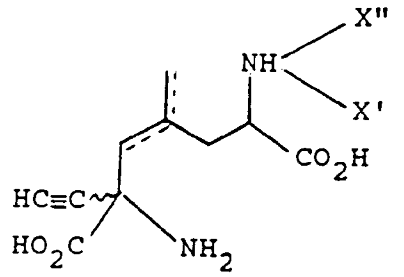

($I_B$)

or the compound of formula ($IV_A$) or ($IV_B$) is subjected to the action of an agent which cleaves the trimethylsilyl in order to obtain the compound of formula ($I_C$):

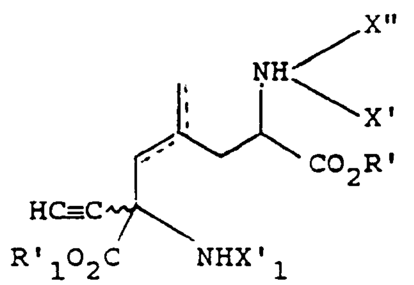

($I_C$)

then if desired, the compound of formula ($I_A$), ($I_B$) or ($I_C$) is subjected to the action of a partial reduction agent in order to obtain the corresponding compound of formula ($I_D$) in which Y represents an ethenyl radical, or to the action of a total reduction agent of the triple bond in order to obtain the corresponding compound of formula ($I_E$) in which Y is an ethyl radical,
then if appropriate the compound of formula (I) thus obtained is subjected to all or one part only of the operations mentioned at the end of claim 13.

**17.** As new chemical products, the compounds of formula (II) as defined in claim 13, in which Y′ is a trimethylsilyl alkynyl radical containing up to 8 carbon atoms.

**18.** As new chemical products, the compounds of formula (III) and ($III_A$) as defined in claims 15 and 16.

**Claims for the following Contracting State : ES**

**1.** Preparation process for the compounds of general formula (I):

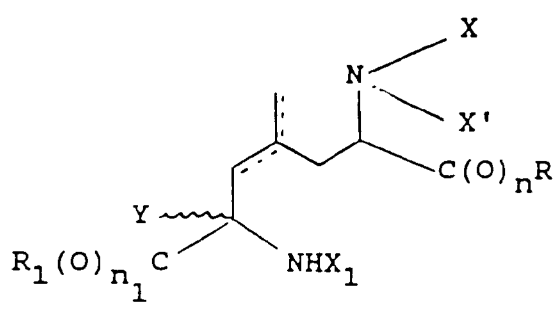

(I)

in which
- the dotted lines represent an optional endo or oxo double bond,
- Y represents an alkyl, alkenyl or alkynyl radical containing 2 to 18 carbon atoms, or an alkyl radical containing 1 to 18 carbon atoms substituted by one or more halogen atoms,
- X, X′ and $X_1$, identical or different, represent a hydrogen atom, an alkyl, alkenyl or alkynyl radical containing up to 8 carbon atoms, an aryl radical containing up to 14 carbon atoms, an aralkyl radical containing up to 18 carbon atoms, or an acyl radical derived from a fatty acid and/or from an alpha and/or omega amino acid,

and either n and/or $n_1$ represent the number 1 and R and/or $R_1$ represent the remainder of an amine or of an alpha or omega amino acid, or n and/or $n_1$ represent the number 2 and R and $R_1$ represent a hydrogen atom, an alkyl radical containing up to 8 carbon atoms or an aryl radical containing up to 14 carbon atoms, or an aralkyl radical containing up to 18 carbon atoms, or a

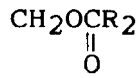

radical, in which $R_2$ represents an alkyl radical containing up to 8 carbon atoms, or an aryl radical containing up to 14 carbon atoms,as well as their salts with organic or mineral acids or with bases, characterized in that a compound of general formula (II):

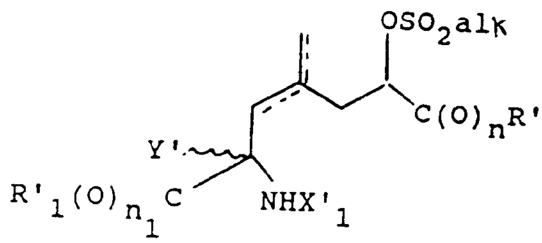

(II)

in which alk represents an alkyl radical containing 1 to 8 carbon atoms optionally substituted by one or more halogen atoms, n, $n_1$ and the dotted lines keep the same meaning as previously, Y′ represents either Y, or a precursor of Y, $X'_1$, R′ and $R'_1$ have the same meaning as $X_1$, R and $R_1$ with the exception of hydrogen, is subjected to the action of a reagent capable of providing a precursor of the amino function or of an amine of formula

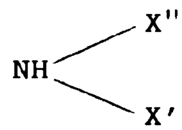

in which X′ has the meaning indicated in claim 1 and X″ has the meaning indicated for X with the exception of hydrogen, in order to obtain, if appropriate after suitable treatment, the compound of formula (I) which is subjected, if desired, to all or one part only of the following operations in any order:

- deprotection of the amine functions,
- functionalization of the amine functions,
- hydrolysis of the ester functions,
- treatment of Y′ in order to obtain Y,
- total or selective reduction of the Y substituent when this is unsaturated,
- salification.

2. Process according to claim 1, for the preparation of the products of formula (I) as defined in claim 1, corresponding to formula (I′):

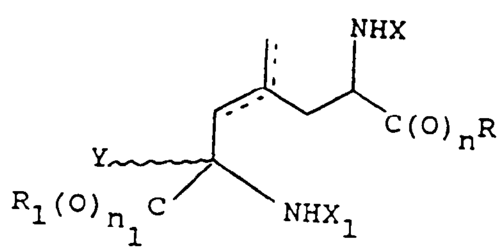

(I′)

in which X, $X_1$, R, $R_1$, n, $n_1$ and Y have the meaning indicated previously, characterized in that a reagent capable of providing a precursor of the amino function or an amine of formula

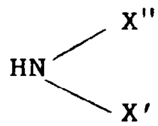

in which X″ has the meaning indicated previously and X′ represents a hydrogen atom, is reacted on the compound of formula (II).

3. Process according to claim 2, characterized in that the reagent capable of providing a precursor of the amino function is sodium nitride, potassium phthalimide or hydroxylamine.

4. Process according to claim 2, characterized in that a compound of formula (II) defined as in claim 13 is subjected to the action of sodium nitride in order to obtain the compound of formula (III):

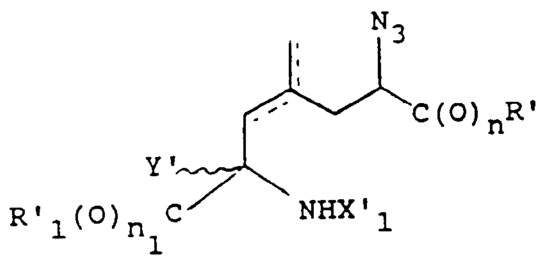

(III)

which is subjected to the action of an agent which reduces the $N_3$ group, in order to obtain the compound of formula (IV):

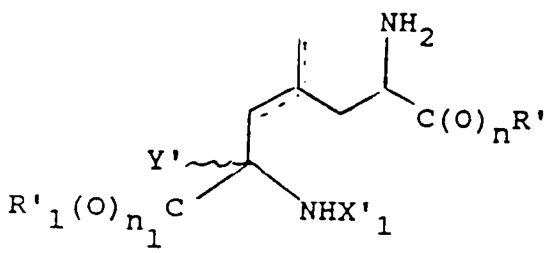

(IV)

which is subjected to all or one part only of the operations mentioned at the end of claim 1.

5. Process according to claim 1, characterized in that a compound of formula ($II_A$):

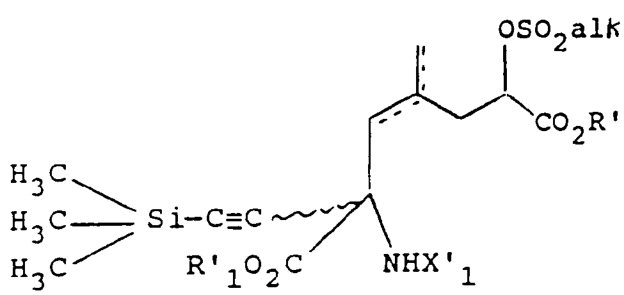

($II_A$)

is subjected

<u>either</u> to the action of sodium nitride in order to obtain the compound of formula ($III_A$):

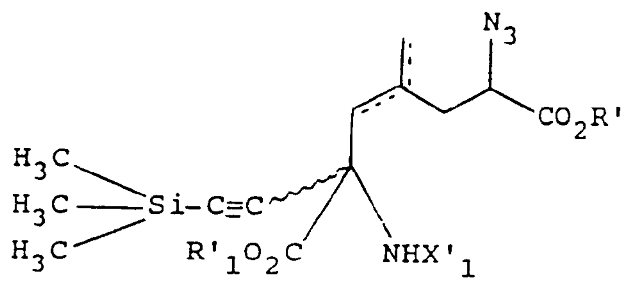

($III_A$)

which is subjected to the action of an agent which reduces the nitride, in order to obtain the compound of formula ($IV_A$):

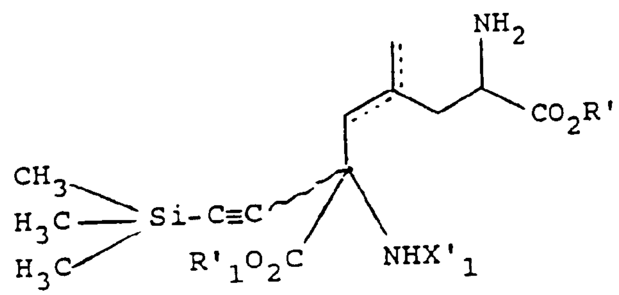

($IV_A$)

or to the action of an amine of formula

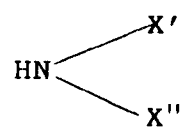

in which X′ and X″ have the meaning indicated in claim 13, in order to obtain the compound of formula ($IV_B$):

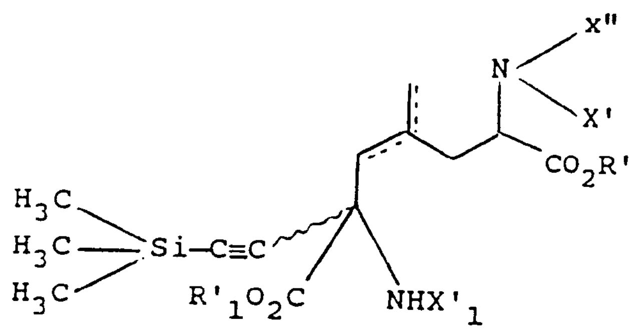

($IV_B$)

then either compound ($IV_A$) or ($IV_B$) is subjected to the action of an agent which selectively cleaves the ester functions and the trimethylsilyl radical in order to obtain the compound of formula ($I_A$):

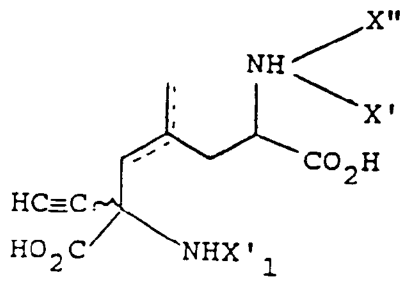

$(I_A)$

which if appropriate is subjected to the action of an agent which cleaves the amine protector group in order to obtain the compound of formula $(I_B)$:

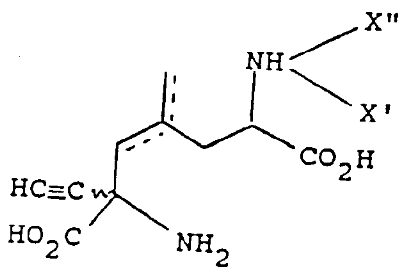

$(I_B)$

or the compound of formula $(IV_A)$ or $(IV_B)$ is subjected to the action of an agent which cleaves the trimethylsilyl in order to obtain the compound of formula $(I_C)$:

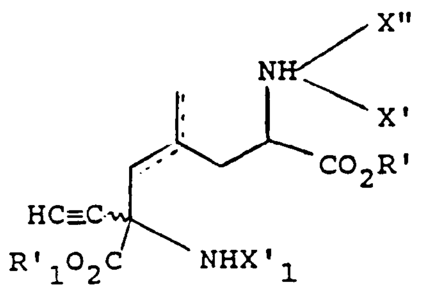

$(I_C)$

then if desired, the compound of formula $(I_A)$, $(I_B)$ or $(I_C)$ is subjected to the action of a partial reduction agent in order to obtain the corresponding compound of formula $(I_D)$ in which Y represents an ethenyl radical, or to the action of a total reduction agent of the triple bond in order to obtain the corresponding compound of formula $(I_E)$ in which Y is an ethyl radical,
then if appropriate the compound of formula (I) thus obtained is subjected to all or one part only of the operations mentioned at the end of claim 1.

6. Process according to claim 5, characterized in that a compound of formula $(II_A)$ as defined in claim 5 is subjected to the action of sodium nitride in order to obtain the compound of formula $(III_A)$ as defined in claim 5, which is subjected to the action of an agent which reduces the nitride in order to obtain the compound of formula $(IV_A)$ as defined in claim 5, then either this compound $(IV_A)$ is subjected to the action of an agent which selectively cleaves the ester functions and the trimethylsilyl radical in order to obtain the compound of formula $(I'_A)$:

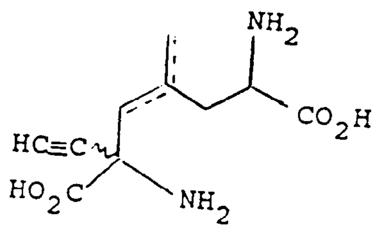

(I'A)

which is appropriate is subjected to the action of an agent which cleaves the amine protector group, in order to obtain the compound of formula ($I'_B$):

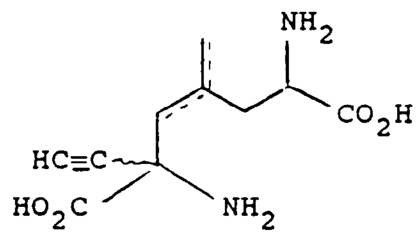

(I'B)

or this compound of formula ($IV_A$) is subjected to the action of an agent which cleaves the trimethylsilyl, in order to obtain the compound of formula ($I'_C$):

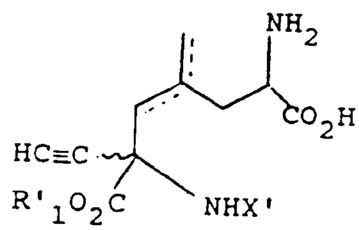

(I'C)

then if desired, the compound of formula ($I'_A$), ($I'_B$) or ($I'_C$) is subjected to the action of a partial reduction agent in order to obtain the corresponding compound of formula ($I'_D$) in which Y represents an ethenyl radical, or to the action of a total reduction agent of the triple bond in order to obtained the corresponding compound of formula ($I'_E$) in which Y is an ethyl radical,
then if desired each of the compounds of formula (I) thus obtained is subjected to all or one part only of the operations mentioned at the end of claim 1.

7. Process according to claim 1, characterized in that a compound of formula (II) is used at the start in which the dotted lines represent an exo double bond.

8. Process according to claim 1, characterized in that an amine of formula

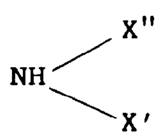

is used at the start in which X' represents a hydrogen atom or a methyl radical and X" represents a methyl or benzyl radical.

9. Process according to claim 1, characterized in that a compound of formula (II) is used at the start in which Y' represents an acetylenic radical or a precursor of an acetylenic radical.

**Claims for the following Contracting State : GR**

1. Preparation process for the compounds of general formula (I):

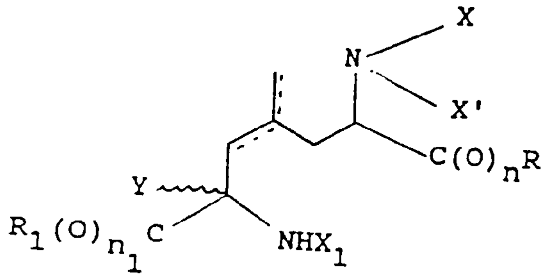

(I)

in which

- the dotted lines represent an optional endo or oxo double bond,
- Y represents an alkyl, alkenyl or alkynyl radical containing 2 to 18 carbon atoms, or an alkyl radical containing 1 to 18 carbon atoms substituted by one or more halogen atoms,
- X, X′ and $X_1$, identical or different, represent a hydrogen atom, an alkyl, alkenyl or alkynyl radical containing up to 8 carbon atoms, an aryl radical containing up to 14 carbon atoms, an aralkyl radical containing up to 18 carbon atoms, or an acyl radical derived from a fatty acid and/or from an alpha and/or omega amino acid,

and <u>either</u> n and/or $n_1$ represent the number 1 and R and/or $R_1$ represent the remainder of an amine or of an alpha or omega amino acid, <u>or</u> n and/or $n_1$ represent the number 2 and R and $R_1$ represent a hydrogen atom, an alkyl radical containing up to 8 carbon atoms or an aryl radical containing up to 14 carbon atoms, or an aralkyl radical containing up to 18 carbon atoms, or a

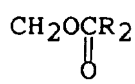

radical, in which $R_2$ represents an alkyl radical containing up to 8 carbon atoms, or an aryl radical containing up to 14 carbon atoms,as well as their salts with organic or mineral acids or with bases, characterized in that a compound of general formula (II):

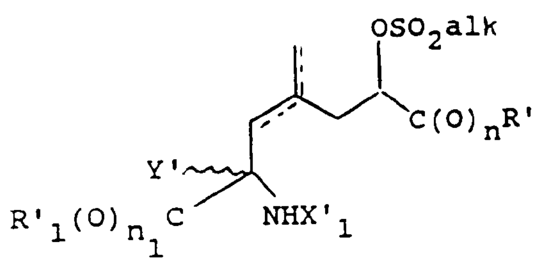

(II)

in which alk represents an alkyl radical containing 1 to 8 carbon atoms optionally substituted by one or more halogen atoms, n, $n_1$ and the dotted lines keep the same meaning as previously, Y′ represents either Y, or a precursor of Y, $X'_1$, R′ and $R'_1$ have the same meaning as $X_1$, R and $R_1$ with the exception of hydrogen, is subjected to the action of a reagent capable of providing a precursor of the amino function or of an amine of formula

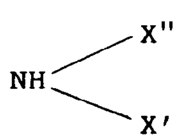

in which X′ has the meaning indicated in claim 1 and X″ has the meaning indicated for X with the exception

of hydrogen, in order to obtain, if appropriate after suitable treatment, the compound of formula (I) which is subjected, if desired, to all or one part only of the following operations in any order:

- deprotection of the amine functions,
- functionalization of the amine functions,
- hydrolysis of the ester functions,
- treatment of Y′ in order to obtain Y,
- total or selective reduction of the Y substituent when this is unsaturated,
- salification.

**2.** Process according to claim 1, for the preparation of the products of formula (I) as defined in claim 1, corresponding to formula (I′):

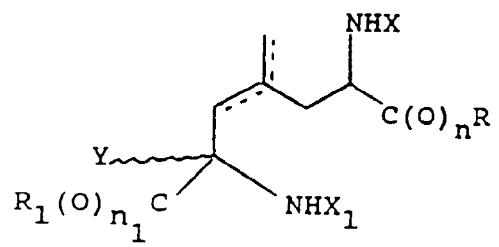

(I′)

in which X, $X_1$, R, $R_1$, n, $n_1$ and Y have the meaning indicated previously, characterized in that a reagent capable of providing a precursor of the amino function or an amine of formula

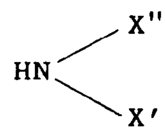

in which X″ has the meaning indicated previously and X′ represents a hydrogen atom, is reacted on the compound of formula (II).

**3.** Process according to claim 2, characterized in that the reagent capable of providing a precursor of the amino function is sodium nitride, potassium phthalimide or hydroxylamine.

**4.** Process according to claim 2, characterized in that a compound of formula (II) defined as in claim 13 is subjected to the action of sodium nitride in order to obtain the compound of formula (III):

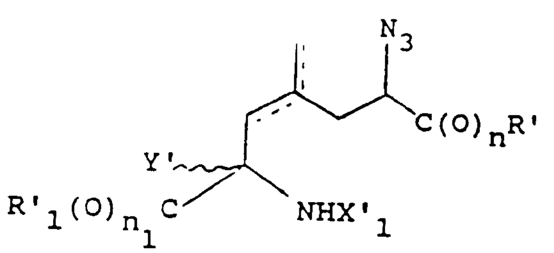

(III)

which is subjected to the action of an agent which reduces the $N_3$ group, in order to obtain the compound of formula (IV):

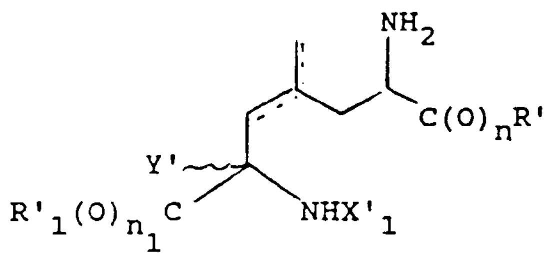

(IV)

which is subjected to all or one part only of the operations mentioned at the end of claim 1.

5. Process according to claim 1, characterized in that a compound of formula ($II_A$):

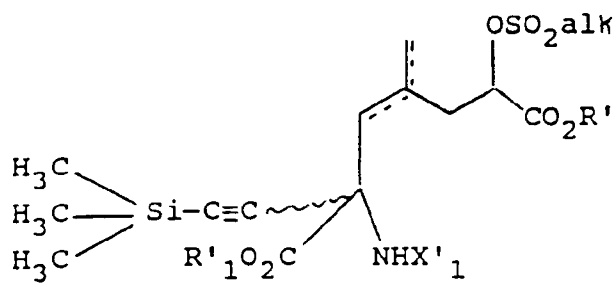

($II_A$)

is subjected

either to the action of sodium nitride in order to obtain the compound of formula ($III_A$):

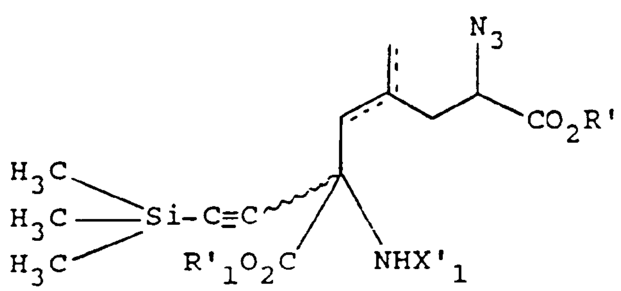

($III_A$)

which is subjected to the action of an agent which reduces the nitride, in order to obtain the compound of formula ($IV_A$):

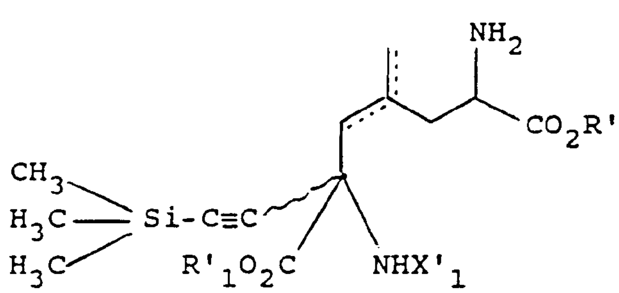

($IV_A$)

or to the action of an amine of formula

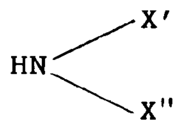

in which X′ and X″ have the meaning indicated in claim 13, in order to obtain the compound of formula ($IV_B$):

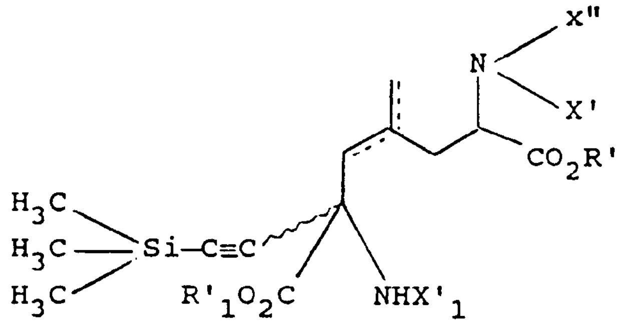

($IV_B$)

then either compound ($IV_A$) or ($IV_B$) is subjected to the action of an agent which selectively cleaves the ester functions and the trimethylsilyl radical in order to obtain the compound of formula ($I_A$):

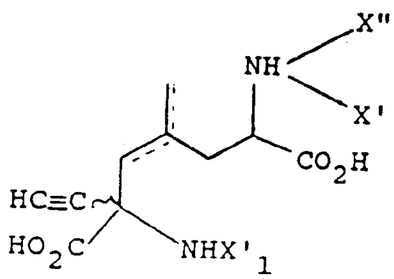

($I_A$)

which if appropriate is subjected to the action of an agent which cleaves the amine protector group in order to obtain the compound of formula ($I_B$):

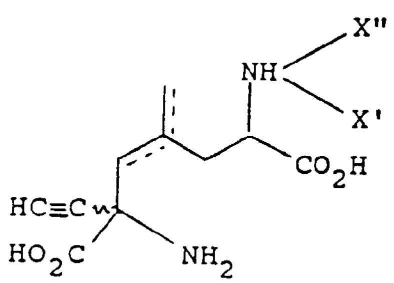

($I_B$)

or the compound of formula ($IV_A$) or ($IV_B$) is subjected to the action of an agent which cleaves the trimethylsilyl in order to obtain the compound of formula ($I_C$):

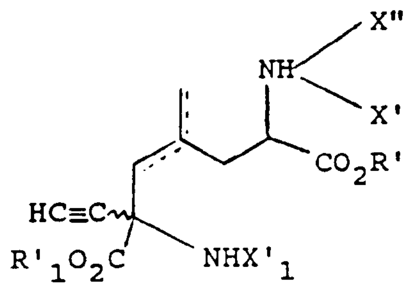

$(I_C)$

then if desired, the compound of formula $(I_A)$, $(I_B)$ or $(I_C)$ is subjected to the action of a partial reduction agent in order to obtain the corresponding compound of formula $(I_D)$ in which Y represents an ethenyl radical, or to the action of a total reduction agent of the triple bond in order to obtain the corresponding compound of formula $(I_E)$ in which Y is an ethyl radical,
then if appropriate the compound of formula (I) thus obtained is subjected to all or one part only of the operations mentioned at the end of claim 1.

6. Process according to claim 5, characterized in that a compound of formula $(II_A)$ as defined in claim 5 is subjected to the action of sodium nitride in order to obtain the compound of formula $(III_A)$ as defined in claim 5, which is subjected to the action of an agent which reduces the nitride in order to obtain the compound of formula $(IV_A)$ as defined in claim 5, then <u>either</u> this compound $(IV_A)$ is subjected to the action of an agent which selectively cleaves the ester functions and the trimethylsilyl radical in order to obtain the compound of formula $(I'_A)$:

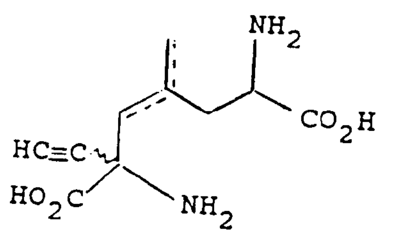

$(I'_A)$

which is appropriate is subjected to the action of an agent which cleaves the amine protector group, in order to obtain the compound of formula $(I'_B)$:

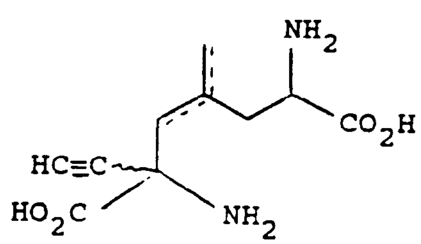

$(I'_B)$

<u>or</u> this compound of formula $(IV_A)$ is subjected to the action of an agent which cleaves the trimethylsilyl, in order to obtain the compound of formula $(I'_C)$:

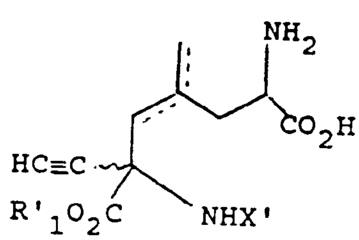

$(I'_C)$

then if desired, the compound of formula ($I'_A$), ($I'_B$) or ($I'_C$) is subjected to the action of a partial reduction agent in order to obtain the corresponding compound of formula ($I'_D$) in which Y represents an ethenyl radical, or to the action of a total reduction agent of the triple bond in order to obtained the corresponding compound of formula ($I'_E$) in which Y is an ethyl radical,
then if desired each of the compounds of formula (I) thus obtained is subjected to all or one part only of the operations mentioned at the end of claim 1.

**7.** Process according to claim 1, characterized in that a compound of formula (II) is used at the start in which the dotted lines represent an exo double bond.

**8.** Process according to claim 1, characterized in that an amine of formula

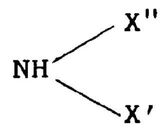

is used at the start in which X′ represents a hydrogen atom or a methyl radical and X″ represents a methyl or benzyl radical.

**9.** Process according to claim 1, characterized in that a compound of formula (II) is used at the start in which Y′ represents an acetylenic radical or a precursor of an acetylenic radical.

**10.** Process according to claim 2, characterized in that any one of the products of formula (I) are prepared, of which the names follow:
- 2,6-diamino 2-ethenyl 4-methylene heptanedioic acid,
- 2,6-diamino 2-ethyl 4-methylene heptanedioic acid,
- 2-amino 2-ethynyl 4-methylene 6-(phenylmethyl) amino heptanedioic acid.

**11.** Preparation process for pharmaceutical compositions, characterized in that at least one of the products of formula (I) as defined in claim 1 or at least one of their salts with pharmaceutically acceptable organic or mineral acids or bases is used as active ingredient in a form intended for this use.

**12.** Preparation process for pharmaceutical compositions, characterized in that at least one of the products of formula (I′) as defined in claim 2 or at least one of their salts with pharmaceutically acceptable organic or mineral acids or bases is used as active ingredient in a form intended for this use.

**13.** Preparation process for pharmaceutical compositions, characterized in that at least one of the products of formula (I) as defined in claim 10 or at least one of their salts with pharmaceutically acceptable organic or mineral acids or bases is used as active ingredient in a form intended for this use.

**14.** As new chemical products, the compounds of formula (II) as defined in claim 1, in which Y′ is a trimethylsilyl alkynyl radical containing up to 8 carbon atoms.

**15.** As new chemical products, the compounds of formulae (III) and ($III_A$) as defined in claims 4 and 5.